# EUROPEAN PATENT SPECIFICATION

(11) **EP 3 131 027 B1**
(45) Date of publication and mention of the grant of the patent: **17.07.2019**
(21) Application number: 16183256.3
(22) Date of filing: 08.08.2016
(51) Int. Cl.: G16H 40/63, G16H 20/30, G16H 50/30, G09B 19/00, H04R 1/10

(54) **METHOD FOR DETECTING ACTIVITY INFORMATION OF USER AND ELECTRONIC DEVICE THEREOF**
VERFAHREN ZUR ERKENNUNG VON AKTIVITÄTSINFORMATIONEN EINES BENUTZERS UND ELEKTRONISCHE VORRICHTUNG DAFÜR
PROCÉDÉ DE DÉTECTION D'INFORMATIONS D'ACTIVITÉ D'UTILISATEUR ET SON DISPOSITIF ÉLECTRONIQUE

(30) Priority: 11.08.2015 KR 20150113288
(43) Date of publication of application: 15.02.2017
(73) Proprietor: Samsung Electronics Co., Ltd., Suwon-si, Gyeonggi-do 16677 (KR)
(72) Inventor: Cho, Junghee, Anyang-si (KR); Ku, Sangchul, Suwon-si (KR); Suh, Jung-Won, Suwon-si (KR); Joo, Sun-Tae, Seoul (KR); Na, Shinyoung, Yongin-si (KR); Kim, Taeho, Cheongju-si (KR); Park, Jeong-Min, Hwaseong-si (KR)
(74) Representative: Nederlandsch Octrooibureau

(56) References cited:
- US-A1- 2008 090 703
- US-A1- 2014 257 535
- US-A1- 2014 293 852
- US-A1- 2015 005 911

## Description

### BACKGROUND

### 1. Field of the Disclosure

The present disclosure relates generally to an electronic device, and more particularly, to an electronic device for detecting activity information of a user.

### 2. Description of the Related Art

With the development of information and communication technologies and semiconductor technologies, various types of electronic devices have developed into multimedia devices that provide various multimedia services. For example, portable electronic devices may provide diverse multimedia services such as broadcast services, wireless Internet services, camera services, and music playback services.

An electronic device may provide a health care service in order to meet the interest in health care of users. For example, an electronic device may provide, to a user, a sequence or method of exercises such as jumping rope and muscular exercises through an exercise application.

When an electronic device provides a health care service, the electronic device may provide a standardized exercise type, exercise sequence, and exercise method. However, such standard exercise type, exercise sequence, and exercise method may not be suitable for all users, i.e., users having various physical conditions. In addition, it may be inconvenient for a user of the electronic device if the user is unable to determine, through an exercise application, whether an exercise has been performed using a correct exercise method corresponding to the user's unique physical condition. Documents US 2008/090703 A1 (ROSENBERG LOUIS B) and US 2014/257535 A1 (MORRIS DANIEL ET AL) disclose exercise tracking systems, representing the closest prior art.

### SUMMARY

According to an aspect of the present disclosure, there is provided a device and method for an exercise application corresponding to activity information of a user by an electronic device, according to the independent claims.

Specific embodiments according to the present disclosure are defined in the dependent claims.

### BRIEF DESCRIPTION OF THE DRAWINGS

The above and other aspects, features, and advantages of certain embodiments of the present disclosure will be more apparent from the following detailed description taken in conjunction with the accompanying drawings, in which:
FIG. 1 is a diagram illustrating a system for providing an exercise service, according to an embodiment of the present disclosure;
FIG. 2 is a diagram illustrating an electronic device within a network environment, according to an embodiment of the present disclosure;
FIG. 3 is a diagram of an electronic device, according to an embodiment of the present disclosure;
FIG. 4 is a diagram of a program module, according to an embodiment of the present disclosure;
FIG. 5 is a graph of a zero-cross algorithm, according to an embodiment of the present disclosure;
FIGs. 6A and 6B are graphs of an energy level algorithm, according to an embodiment of the present disclosure;
FIGs. 7A and 7B are graphs of a vertical movement algorithm, according to an embodiment of the present disclosure;
FIG. 8 is a flow-chart of a method for providing guide information based on activity information of a user by an electronic device, according to an embodiment of the present disclosure;
FIG. 9 is a flow-chart of a method for selecting activity schedule information corresponding to environmental information by an electronic device, according to an embodiment of the present disclosure;
FIG. 10 is a flow-chart of a method for receiving, from an external device, activity schedule information by an electronic device, according to an embodiment of the present disclosure;
FIG. 11 is a flow-chart of a method for acquiring activity schedule information by an electronic device, according to an embodiment of the present disclosure;
FIG. 12 is a flow-chart of a method for acquiring activity information of a user by an electronic device, according to an embodiment of the present disclosure;
FIG. 13 is a flow-chart of a method for updating activity schedule information by an electronic device, according to an embodiment of the present disclosure;
FIG. 14 is a flow-chart of a method for updating activity schedule information by an electronic device, according to an embodiment of the present disclosure;
FIG. 15 is a signal diagram of a method for collecting activity information of a user, according to an embodiment of the present disclosure;
FIGs. 16A-FIG. 16H are diagrams of screen configurations for providing an exercise service by an electronic device, according to an embodiment of the present disclosure;
FIG. 17 is a flow-chart of a method for controlling an exercise service of an external device by an electronic device, according to an embodiment of the present disclosure; and
FIG. 18 is a flow-chart of a method for providing an exercise service, based on a control of an external device, by an electronic device, according to an embodiment of the present disclosure.

### DETAILED DESCRIPTION

The scope of the invention is defined by the claims. The following embodiments merely define examples, only those falling within the scope of the claims are part of the invention.

The terms "have," "may have," "include," and "may include" as used herein indicate the presence of corresponding features (for example, elements such as numerical values, functions, operations, or parts), and do not preclude the presence of additional features.

The terms "A or B," "at least one of A or/and B," or "one or more of A or/and B" as used herein include all possible combinations of items enumerated with them. For example, "A or B," "at least one of A and B," or "at least one of A or B" means (1) including at least one A, (2) including at least one B, or (3) including both at least one A and at least one B.

The terms such as "first" and "second" as used herein may modify various elements regardless of an order and/or importance of the corresponding elements, and do not limit the corresponding elements. These terms may be used for the purpose of distinguishing one element from another element. For example, a first user device and a second user device may indicate different user devices regardless of the order or importance. For example, a first element may be referred to as a second element without departing from the scope the present invention, and similarly, a second element may be referred to as a first element.

It will be understood that, when an element (for example, a first element) is "(operatively or communicatively) coupled with/to" or "connected to" another element (for example, a second element), the element may be directly coupled with/to another element, and there may be an intervening element (for example, a third element) between the element and another element. To the contrary, it will be understood that, when an element (for example, a first element) is "directly coupled with/to" or "directly connected to" another element (for example, a second element), there is no intervening element (for example, a third element) between the element and another element.

The expression "configured to (or set to)" as used herein may be used interchangeably with "suitable for," "having the capacity to," "designed to," " adapted to," "made to," or "capable of' according to a context. The term "configured to (set to)" does not necessarily mean "specifically designed to" in a hardware level. Instead, the expression "apparatus configured to..." may mean that the apparatus is "capable of..." along with other devices or parts in a certain context. For example, "a processor configured to (set to) perform A, B, and C" may mean a dedicated processor (e.g., an embedded processor) for performing a corresponding operation, or a generic-purpose processor (e.g., a CPU or an application processor) capable of performing a corresponding operation by executing one or more software programs stored in a memory device.
The term "module" as used herein may be defined as, for example, a unit including one of hardware, software, and firmware or two or more combinations thereof. The term "module" may be interchangeably used with, for example, the terms "unit", "logic", "logical block", "component", or "circuit", and the like. The "module" may be a minimum unit of an integrated component or a part thereof. The "module" may be a minimum unit performing one or more functions or a part thereof. The "module" may be mechanically or electronically implemented. For example, the "module" may include at least one of an application-specific integrated circuit (ASIC) chip, field-programmable gate arrays (FPGAs), or a programmable-logic device, which is well known or will be developed in the future, for performing certain operations.

The terms used in describing the various embodiments of the present disclosure are for the purpose of describing particular embodiments and are not intended to limit the present disclosure. As used herein, the singular forms are intended to include the plural forms as well, unless the context clearly indicates otherwise. All of the terms used herein including technical or scientific terms have the same meanings as those generally understood by an ordinary skilled person in the related art unless they are defined otherwise. The terms defined in a generally used dictionary should be interpreted as having the same or similar meanings as the contextual meanings of the relevant technology and should not be interpreted as having ideal or exaggerated meanings unless they are clearly defined herein. According to circumstances, even the terms defined in this disclosure should not be interpreted as excluding the embodiments of the present disclosure.

An electronic device according to various embodiments of the present disclosure, for example, may include at least one of a smartphone, a tablet personal computer (PC), a mobile phone, a video phone, an electronic book (e-book) reader, a desktop PC, a laptop PC, a netbook computer, a workstation, a server, a personal digital assistant (PDA), a portable multimedia player (PMP), an MP3 player, a mobile medical appliance, a camera, and a wearable device (e.g., smart glasses, a head-mounted-device (HMD), electronic clothes, an electronic bracelet, an electronic necklace, an electronic appcessory, an electronic tattoo, a smart mirror, or a smart watch).

The electronic device may be a smart home appliance. The home appliance may include at least one of, for example, a television, a Digital Video Disk (DVD) player, an audio, a refrigerator, an air conditioner, a vacuum cleaner, an oven, a microwave oven, a washing machine, an air cleaner, a set-top box, a home automation control panel, a security control panel, a TV box (e.g., Samsung HomeSync™, Apple TV™, or Google TV™), a game console (e.g., Xbox™ and PlayStation™), an electronic dictionary, an electronic key, a camcorder, and an electronic photo frame.

The electronic device may include at least one of various medical devices (e.g., various portable medical measuring devices (a blood glucose monitoring device, a heart rate monitoring device, a blood pressure measuring device, a body temperature measuring device, etc.), a magnetic resonance angiography (MRA), a magnetic resonance imaging (MRI), a computed tomography (CT) machine, and an ultrasonic machine), a navigation device, a global positioning system (GPS) receiver, an event data recorder (EDR), a flight data recorder (FDR), a vehicle infotainment devices, an electronic devices for a ship (e.g., a navigation device for a ship, and a gyro-compass), avionics devices, security devices, an automotive head unit, a robot for home or industry, an automatic teller's machine (ATM) in banks, point of sales (POS) devices in a shop, or an Internet of Things (IoT) device (e.g., a light bulb, various sensors, electric or gas meter, a sprinkler device, a fire alarm, a thermostat, a streetlamp, a toaster, a sporting goods, a hot water tank, a heater, a boiler, etc.).

The electronic device may include at least one of a part of furniture or a building/structure, an electronic board, an electronic signature receiving device, a projector, and various kinds of measuring instruments (e.g., a water meter, an electric meter, a gas meter, and a radio wave meter). The electronic device may be a combination of one or more of the aforementioned various devices. The electronic device may be a flexible device. Further, the electronic device is not limited to the aforementioned devices, and may include a new electronic device according to the development of new technologies.

Hereinafter, an electronic device will be described with reference to the accompanying drawings. As used herein, the term "user" may indicate a person who uses an electronic device or a device (e.g., an artificial intelligence electronic device) that uses an electronic device.

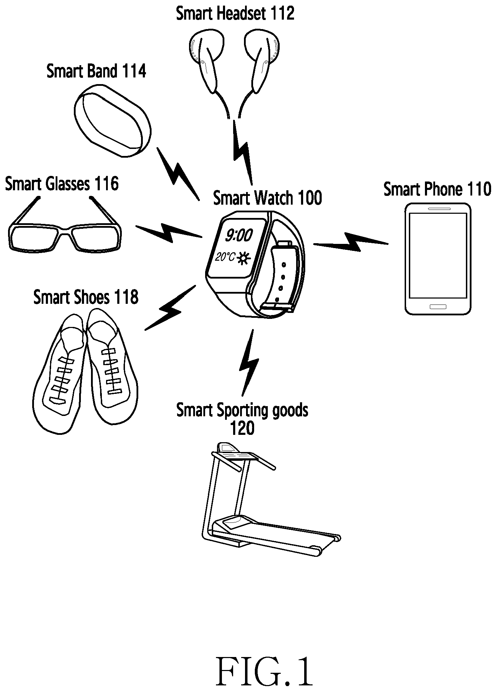

FIG. 1 is a diagram of a system for providing an exercise service, according to an embodiment of the present disclosure.

Referring to FIG. 1, a system for providing an exercise service may include electronic devices 100, 112 to 118 that are worn on the body of a user or an electronic device 110 that is not worn on the body of a user. According to an embodiment, the electronic device that is worn on the body of a user may include a smart watch 100, a smart headset 112, a smart band 114, smart glasses 116, and a smart shoe 118. In addition, the system for providing an exercise service may include an exercise equipment (smart sporting goods) 120 having one or more sensors (e.g., a treadmills).

The electronic devices 100 to 120 may detect activity information of a user through one or more sensors. For example, the sensor may include a 9-axis sensor, a barometer, a heart rate sensor, a pressure sensor, a global navigation satellite system (GNSS) sensor, a communication module, and so on. For example, the 9-axis sensor may be referred to as a motion sensor, and include at least one of an acceleration sensor, a gyroscope, and a geomagnetic sensor. Activity information of a user according to various embodiments may include changes in body movements (e.g., gestures, moving a location, etc.) and biometric information (e.g., heart rate, oxygen saturation, burned calories, etc.), the location of a user, or the like.

Each of the electronic devices 100 to 120 may collect activity information of a user through one or more sensors provided in the electronic device. For example, the smart watch 100, the smart phone 110, the smart headset 112, and the smart band 114 may include a 9-axis sensor, a barometer, and a heart rate sensor. The smart glasses 116 may include a 9-axis sensor and a barometer. The smart shoe 118 may include a 9-axis sensor, a barometer, and a pressure sensor.

The electronic devices 100 to 120 may collect activity information of a user, using one or more peripheral devices 100 to 120. For example, the electronic devices 100 to 120 have recognizable activity information of a user, which may be different according to a location attached to or in contact with the body of the user, or the type of a sensor included in the electronic devices 100 to 120. Accordingly, the electronic devices may interwork with one or more peripheral devices to correspond to the exercise type of the user and sensor information of the electronic devices 100 to 120 so as to detect activity information of the user. For example, the electronic device (e.g., a smart watch 100) may be connected to communicate with one or more peripheral devices among the smart phone 110, the smart headset 112, the smart band 114, the smart glasses 116, the smart shoe 118, and the smart sporting goods 120. The electronic device (e.g., a smart watch 100) may interwork with one or more peripheral devices connected to communicate therewith so as to collect user activity information. For example, the smart watch 100 may be connected to and communicate with a peripheral device through a short range communication scheme such as Wi-Fi, Bluetooth, Bluetooth low energy (BLE), and near field communication (NFC).

The electronic devices may analyze activity information of a user acquired through the electronic device or one or more peripheral devices 100 to 120 so as to generate and output exercise guide information. For example, the exercise guide information may include exercise state related information, exercise start information, posture correction information, exercise schedule related information, exercise schedule change information, and so on.

Each of the electronic devices 100 to 120 may output exercise guide information through at least one output module. The smart watch 100, the smart phone 110, and the smart glasses 116 according to various embodiments may include a speaker, a display, an LED indicator, and a vibration sensor. The smart headset 112 according to various embodiments may include a speaker. The smart band 114 according to various embodiments may include a display and a vibration sensor.

The electronic devices may output posture correction information, based on information of a location which each of the peripheral devices is attached to or in contact with. For example, the electronic devices may output posture correction information that instructs to change the location of a hand which the smart watch 100 or the smart band 114 is worn towards a first direction (e.g., left side) by a first angle. For example, the electronic devices may output posture correction information that instructs to additionally move the angle of a leg having a foot on which the smart shoe 118 is worn towards a second direction (e.g., upper direction) by a second angle. For example, the electronic devices may output posture correction information that instructs to change the location of an arm in contact with smart sporting goods 120 (e.g., pec deck fly machine) towards a third direction (e.g., downward direction). For example, the electronic device may output posture correction information in the form of s graphic or voice through the electronic device or one or more peripheral devices.

The electronic devices may output exercise guide information using one or more peripheral devices. For example, the electronic devices 100 to 120 have recognition rates of a user for output information of each of the electronic devices 100 to 120, which may be different according to the location on the body which the electronic devices are attached to or in contact with and the type of an output module included in the electronic devices 100 to 120. Accordingly, the electronic devices may interwork with one or more peripheral devices to correspond to characteristics of output information (e.g., auditory sense, vision, tactile sensation, etc.) and output module information of the electronic devices 100 to 120 so as to output exercise guide information. For example, the smart watch 100 may perform a control to output at least one of exercise start information, exercise type change information, and posture correction information, through the speaker of the smart headset 112. For example, the smart phone 110 may perform a control to output at least one of exercise start information, exercise type change information, and posture correction information, through the speaker and display of the smart glasses 116.

The electronic devices may output exercise guide information through one or more output devices (e.g., an IoT device) connectable to communicate therewith. For example, the electronic devices 100 to 120 may be connected to communicate with one or more output devices (e.g., a TV, a monitor, a speaker, etc.). The electronic devices 100 to 120 may output exercise guide information to one or more output devices connected thereto for communication. For example, the smart watch 100 may be connected to communicate with a neighboring TV so as to output exercise guide information. The electronic devices according to various embodiments may be connected to one or more output devices through a short range communication scheme or connected to a server.

The electronic devices 100 to 120 may provide an exercise service through a communication connection with a server. For example, the smart watch 100 may receive activity schedule information from the server. For example, activity schedule information may include an exercise program including the number of times of exercise or a type of exercise, exercise place information, exercise schedule or the like.

The smart headset 112 may output a sound signal, and measure at least one of a user's movement and a user's biometric information, using one or more sensors. According to an embodiment, since the smart headset 112 is put on a part (ear) of a user's body and is able to output exercise guide information to the user, the smart headset 112 may be responsible for progressing an exercise service in a noisy environment in which crowds are gathered. For example, the smart headset 112 may output a sound to guide exercise, which is provided by an external device (e.g., a smart watch 100) or the smart phone 110. For example, the smart headset 112 may output a simple sound (e.g., a beep sound) corresponding to exercise guide information provided by an external device.

The electronic devices including a display and an input means may function as a control device that provides an exercise service using multiple peripheral devices. For example, the control device may analyze partial user activity information (raw data) acquired through each of peripheral devices according to an exercise type, so as to obtain various exercise results generated according to exercise movements such as the number of times of exercise, posture, intensity, and tremors. The control device may perform a control to generate or update exercise guide information corresponding to the exercise results and output the exercise guide information through output modules of the peripheral devices. For example, the control device may collect a partial exercise result acquired through each of the peripheral devices according to an exercise type, so as to acquire various exercise results generated according to exercise movements. For example, each of the peripheral devices may be equipped with a unique and organic exercise engine and derive an exercise result based on user activity information, so as to transmit the example result to the control device. For example, the electronic devices including a display and an input means may include the smart watch 100, the smart phone 110, the smart glasses 116, or the like.

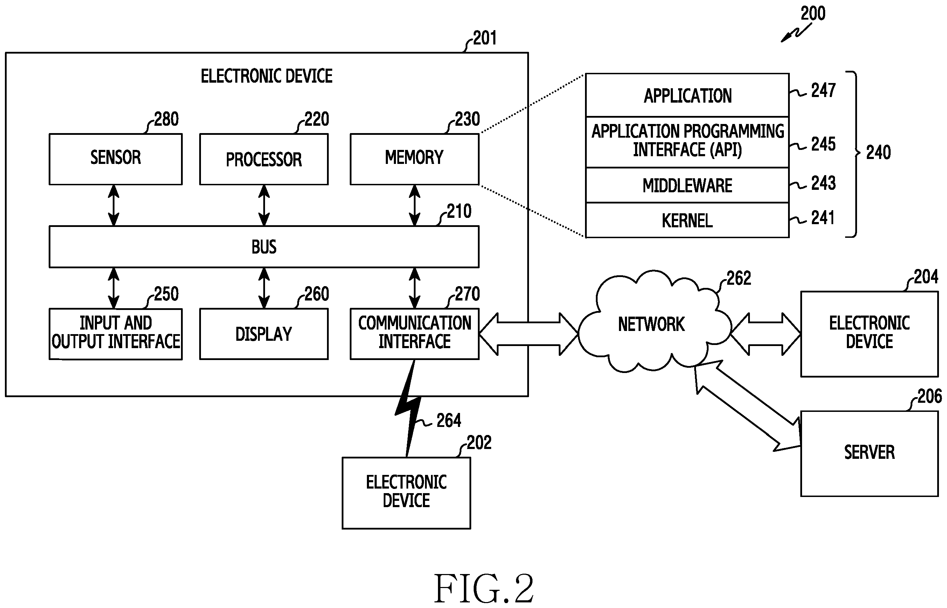

FIG. 2 is a diagram of an electronic device 201 within a network environment 200, according to an embodiment of the present disclosure. According to various embodiments, the electronic device 201 may be one of configurations of a smart watch 100, a smart phone 110, and smart glasses 116, in FIG. 1.

Referring to FIG. 2, an electronic device 201 may include a bus 210, a processor 220 (e.g., including processing circuitry), a memory 230, an input/output interface 250 (e.g., including input/output circuitry), a display 260 (e.g., including display circuitry), a communication interface 270 (e.g., including communication circuitry), and a sensor 280 (e.g., including sensor circuitry). In some embodiments, the electronic device 201 may omit at least one of the above elements or further include other elements.

The bus 210 may include, for example, a circuit that interconnects components 220 to 280 and transmits communication (e.g., a control message and/or data) among components 220 to 280.

The processor 220 may include one or more of a central processing unit (CPU), an application processor (AP), and a communication processor (CP). The processor 220, for example, may perform an operation or data processing relating to control and/or communication of one or more other elements of the electronic device 201.

The processor 220 may perform a control to provide an exercise service based on activity schedule information (e.g., an exercise program). For example, the processor 220 may perform a control to provide an exercise service based on an exercise program received by a server 206 through the communication interface 270. The server 206 according to various embodiments may be a health care server or an exercise service providing server. For example, the server 206 may be a server operated by a predetermined health care service provider. According to various embodiments, activity schedule information may be generated (e.g., written) through an on-line or off-line interview between a trainer of the health care service provider (e.g., an exercise manager) and a user of the electronic device 201.

The processor 220 may perform a control to provide an exercise service based on an exercise program stored in the memory 230. For example, the processor 220 may select an exercise program corresponding to environmental information of the electronic device 201 in the memory 230. For example, the environmental information of the electronic device 201 may include at least one of the location of the electronic device 201, weather, and time.

The processor 220 may perform a control to provide an exercise service based on an exercise program input through the input/output interface 250. According to various embodiments, the processor 220 may control the communication interface 270 to transmit exercise program information to one or more peripheral devices (e.g., external electronic devices 202 and 204) for interworking an exercise service.

The processor 220 may detect activity information of a user through the sensor 280, based on activity schedule information (e.g., an exercise program). For example, the processor 220 may select one or more recognition algorithms corresponding to activity schedule information (e.g., an exercise program) among multiple recognition algorithms. The processor 220 may detect activity information of a user, using one or more sensors corresponding to one or more recognition algorithms among multiple sensors included in the sensor 280. For example, the activity information of a user may include at least one of movement information of a user or biometric information of user.

The processor 220 may detect activity information of a user through one or more peripheral devices (e.g., external electronic devices 202 and 204), based on activity schedule information (e.g., an exercise program). For example, the processor 220 may check a sensor list of peripheral devices connected thereto for communication. The processor 220 may select one or more recognition algorithms corresponding to activity schedule information (e.g., an exercise program) among multiple recognition algorithms. The processor 220 may determine a peripheral device for collecting activity information, based on one or more sensors corresponding to one or more recognition algorithms and a sensor list of the peripheral device. The processor 220 may transmit a request signal for collecting activity information to the peripheral devices for collecting activity information through the communication interface 270. The processor 220 may receive activity information of a user through the communication interface 270 by one or more peripheral devices. For example, the request signal for collecting activity information may include sensor information to be used for collecting activity information by the peripheral devices or recognition algorithm information.

The processor 220 may perform a control to output exercise guide information corresponding to activity information of a user acquired through at least one of the sensor 280 and the peripheral devices. For example, the processor 220 may compare the exercise guide information corresponding to activity information of a user acquired through at least one of the sensor 280 and the peripheral devices with an exercise program so as to generate exercise guide information. The processor 220 may control at least one of the speaker and display 260 of the input/output interface 250 to output the exercise guide information. For example, the processor 220 may check an output module list of peripheral devices connected for communication. The processor 220 may select one or more peripheral devices to interwork for an exercise guide information output, based on the output module list of the peripheral devices and output characteristics of exercise guide information (e.g., auditory sense, vision, tactile sensation, etc.). The processor 220 may transmit the exercise guide information to the one or more peripheral devices to interwork for the exercise guide information output through the communication interface 270.

The memory 230 may include a volatile memory and/or a non-volatile memory. The memory 230, for example, may store a command relating to at least one of other elements of the electronic device 201, or data (e.g., exercise program information, recognition algorithm information). According to an embodiment, the memory 230 may store software and/or a program 240. For example, the program may include a kernel 241, middleware 243, an application programming interface (API) 245, an application program (or application) 247, or the like. At least some of the kernel 241, middleware 243, and API 245 may be referred to as an operating system (OS).

The input/output interface 250 may function as, for example, an interface that may transfer instructions or data input from a user or another external device to the other element(s) of the electronic device 201. In addition, the input/output interface 250 may output instructions or data received from other element(s) of the electronic device 201 to the user or another external device. For example, the input/output interface 250 may include an audio processing module and speaker for outputting a sound signal.

The display 260 may display, for example, various types of contents (e.g., text, images, videos, icons, symbols, or the like) for the user. The display 260 may include a touch screen and receive, for example, a touch, gesture, proximity, or hovering input by using an electronic pen or the user's body part.

The communication interface 270 may set communication between, for example, the electronic device 201 and an external device (e.g., a first external electronic device 202, a second external electronic device 204, or a server 206). For example, the communication interface 270 may be connected to a network 262 through wireless or wired communication to communicate with the external device (e.g., the second external electronic device 204 or the server 206). For example, the communication interface 270 may communicate with the external device (e.g., a first external electronic device 202) through a short range communication scheme 264.

The network 262 may include at least one of a communication network such as a computer network (e.g., a LAN or a WAN), the Internet, and a telephone network.

Each of the first and second external electronic devices 202 and 204 may be of a type identical to or different from that of the electronic device 201. According to an embodiment, the server 206 may include a group of one or more servers. According to various embodiments, all or some of the operations performed in the electronic device 201 may be performed in another electronic device or multiple electronic devices 202, 204 or the server 206. According to an embodiment, when the electronic device 201 has to perform some functions or services automatically or in response to a request, the electronic device 201 may make a request for performing at least some functions relating thereto to another device 202, 204 or the server 206 instead of performing the functions or services by itself or in addition. Another electronic device or the server 206 may execute the requested functions or the additional functions, and may deliver a result of the execution to the electronic device 201. The electronic device 201 may provide the received result as it is or additionally process the result and provide the requested functions or services. To achieve this, for example, cloud computing, distributed computing, or client-server computing technology may be used.

The sensor 280 may acquire activity information of a user. For example, the sensor 280 may include a 9-axis sensor, a barometer, a heart rate sensor, a pressure sensor, and so on. For example, the 9-axis sensor may be referred to as a motion sensor, and include at least one of an acceleration sensor, a gyroscope, and a geomagnetic sensor.

The electronic device 201 may interwork with the external device for an exercise service. According to an embodiment, the processor 220 may control the communication interface 270 to transmit, to the external device, activity information of a user detected through the sensor 280. For example, the processor 220 may select one or more sensors for collecting activity information among multiple sensors included in the sensor 280, based on a request signal for collecting activity information provided by the external device. The processor 220 may collect activity information of a user through one or more sensors. The processor 220 may transmit the activity information of a user to the external device through the communication interface 270. In addition, the processor 220 may derive an exercise result based on the activity information of a user. The processor 220 may transmit the exercise result to the external device through the communication interface 270.

The processor 220 may perform a control to output exercise guide information provided by an external device. For example, the processor 220 may control the speaker or display 260 of the input/output interface 250 to output the exercise guide information provided by the external device.

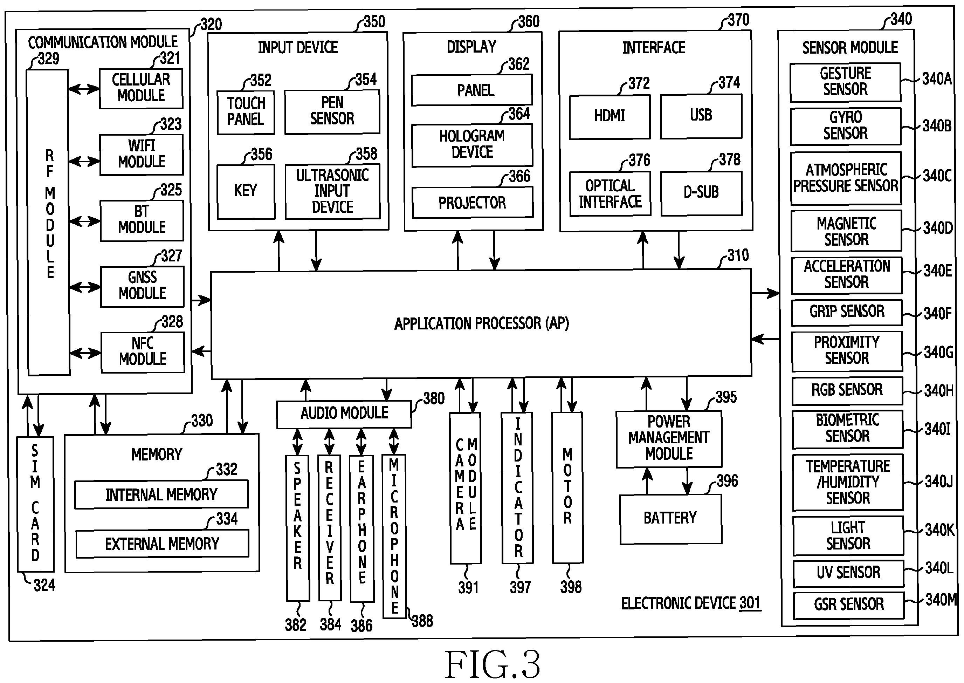

FIG. 3 is a diagram of an electronic device 301, according to an embodiment of the present disclosure. The electronic device 301 may constitute, for example, the entirety or a part of the electronic device 201 illustrated in FIG. 2. The electronic device 301 may include at least one application processor (AP) 310, a communication module 320, a subscriber identification module (SIM) card 324, a memory 330, a sensor module 340, an input device 350, a display 360, an interface 370, an audio module 380, a camera module 391, a power management module 395, a battery 396, an indicator 397, and a motor 398.

The processor 310 may control multiple hardware or software components connected to the processor 310 by driving an operating system or an application program and perform processing of various pieces of data and calculations. The processor 310 may be implemented by, for example, a system on chip (SoC). The processor 310 may further include a graphic processing unit (GPU) and/or an image signal processor. The processor 310 may include at least some (e.g., the cellular module 321) of the elements illustrated in FIG. 3. The processor 310 may load, into a volatile memory, instructions or data received from at least one (e.g., a non-volatile memory) of the other elements and may process the loaded instructions or data, and may store various data in a non-volatile memory.

The processor 310 may perform a control to provide an exercise service based on activity information of a user detected through one or more peripheral devices or the sensor module 340.

The communication module 320 may have a configuration equal or similar to that of the communication interface 270 of FIG. 2. The communication module 320 may include, for example, a cellular module 321, a wireless fidelity (Wi-Fi) module 323, a Bluetooth module 325, a GNSS module 327 (e.g., a GPS module, a Glonass module, a Beidou module, or a Galileo module), an NFC module 328, and a Radio Frequency (RF) module 329.

The cellular module 321 may provide a voice call, an image call, a text message service, or an Internet service through, for example, a communication network. The cellular module 321 may identify and authenticate the electronic device 301 within a communication network using the SIM card 324. The cellular module 321 may perform at least some of the functions that the processor 310 may provide. The cellular module 321 may include a Communication Processor (CP).

The Wi-Fi module 323, the Bluetooth module 325, the GNSS module 327, or the NFC module 328 may include, for example, a processor that processes data transmitted and received through the corresponding module. At least some (e.g., two or more) of the cellular module 321, the Wi-Fi module 323, the Bluetooth module 325, the GNSS module 327, and the NFC module 328 may be included in one Integrated Chip (IC) or IC package.

The RF module 329 may transmit/receive, for example, a communication signal (e.g., an RF signal). At least one of the cellular module 321, the Wi-Fi module 323, the Bluetooth module 325, the GNSS module 327, and the NFC module 328 may transmit and receive RF signals through a separate RF module.

The SIM 324 may be an embedded SIM, and may contain unique identification information (e.g., an Integrated Circuit Card Identifier (ICCID)) or subscriber information (e.g., an International Mobile Subscriber Identity (IMSI)).

The memory 330 (e.g., the memory 230) may include, for example, an embedded memory 332 or an external memory 334. The external memory 334 may be functionally and/or physically connected to the electronic device 301 through various interfaces.

The sensor module 340 may measure a physical quantity or detect an operation state of the electronic device 301, and may convert the measured or detected information into an electrical signal. The sensor module 340 may include, for example, at least one of a gesture sensor 340A, a gyro sensor 340B, an atmospheric pressure sensor 340C, a magnetic sensor 340D, an acceleration sensor 340E, a grip sensor 340F, a proximity sensor 340G, a color sensor 340H (e.g., a red, green, blue (RGB) sensor), a biometric sensor 3401, a temperature/humidity sensor 340J, a light sensor 340K, and a ultraviolet (UV) sensor 340M. Additionally or alternatively, the sensor module 340 may include, for example, an E-nose sensor, an electromyography (EMG) sensor, an electroencephalogram (EEG) sensor, an electrocardiogram (ECG) sensor, an Infrared (IR) sensor, an iris sensor, and/or a fingerprint sensor. The sensor module 340 may further include a control circuit for controlling one or more sensors included therein. In some embodiments, the electronic device 301 may further include a processor configured to control the sensor module 340 as a part of or separately from the processor 310, and may control the sensor module 340 while the processor 310 is in a sleep state.

The input device 350 may include, for example, a touch panel 352, a (digital) pen sensor 354, a key 356, and an ultrasonic input unit 358. The touch panel 352 may use at least one of, for example, a capacitive type, a resistive type, an infrared type, and an ultrasonic type. Also, the touch panel 352 may further include a control circuit. The touch panel 352 may further include a tactile layer and provide a tactile reaction to the user.

The (digital) pen sensor 354 may include, for example, a recognition sheet which is a part of the touch panel or is separated from the touch panel. The key 356 may include, for example, a physical button, an optical key or a keypad. The ultrasonic input device 358 may detect ultrasonic waves generated by an input tool through a microphone 388 and identify data corresponding to the detected ultrasonic waves.

The display 360 (e.g., the display 260) may include a panel 362, a hologram device 364, or a projector 366. The panel 362 may include a configuration identical or similar to that of the display 260 illustrated in FIG. 2. The panel 362 may be implemented to be, for example, flexible, transparent, or wearable. The panel 362 and the touch panel 352 may be implemented as one module. The hologram 364 may show a three dimensional image in the air by using an interference of light. The projector 366 may display an image by projecting light onto a screen. The screen may be located, for example, inside or outside the electronic device 301. According to an exemplary embodiment, the display 360 may further include a control circuit for controlling the panel 362, the hologram device 364, or the projector 366.

The interface 370 may include, for example, a high-definition multimedia interface (HDMI) 372, a universal serial bus (USB) 374, an optical interface 376, or a D-subminiature (D-sub) 378. The interface 370 may be included in, for example, the communication interface 270 illustrated in FIG. 2. Additionally or alternatively, the interface 370 may, for example, include a mobile high-definition link (MHL) interface, a secure digital (SD) card/multi-media card (MMC) interface, or an infrared data association (IrDA) interface.

The audio module 380 may bilaterally convert, for example, a sound and an electrical signal. At least some elements of the audio module 380 may be included in, for example, the input/output interface 250 illustrated in FIG. 2. The audio module 380 may process sound information that is input or output through, for example, a speaker 382, a receiver 384, earphones 386, the microphone 388, or the like.

The camera module 391 is a device which may photograph a still image and a dynamic image. The camera module 291 may include one or more image sensors (e.g., a front sensor or a back sensor), a lens, an Image Signal Processor (ISP) or a flash (e.g., LED or xenon lamp).

The power management module 395 may manage, for example, the power of the electronic device 301. The power management module 395 may include a power management integrated Circuit (PMIC), a charger Integrated Circuit (IC), or a battery gauge. The PMIC may have a wired and/or wireless charging scheme. Examples of the wireless charging method may include, for example, a magnetic resonance method, a magnetic induction method, an electromagnetic wave method, and the like. Additional circuits (e.g., a coil loop, a resonance circuit, a rectifier, etc.) for wireless charging may be further included. The battery gauge may measure, for example, a residual quantity of the battery 396, and a voltage, a current, or a temperature during the charging.

The battery 396 may include, for example, a rechargeable battery and/or a solar battery. According to an embodiment, the battery 396 may include multiple cells that can be connected in series or in parallel.

The indicator 397 may display a particular state (e.g., a booting state, a message state, a charging state, or the like) of the electronic device 301 or a part (e.g., the processor 310) of the electronic device 301. The motor 398 may convert an electrical signal into mechanical vibration, and may generate vibration, a haptic effect, or the like. Although not illustrated, the electronic device 301 may include a processing unit (e.g., a GPU) for supporting a mobile television (TV). The processing unit for supporting mobile TV may, for example, process media data according to a certain standard such as digital multimedia broadcasting (DMB), digital video broadcasting (DVB), or mediaFlo™.

Each of the above-described component elements of hardware according to the present disclosure may be configured with one or more components, and the names of the corresponding component elements may vary based on the type of electronic device. The electronic device of the present disclosure may include at least one of the aforementioned elements. Some elements may be omitted or other additional elements may be further included in the electronic device. Also, some of the hardware components according to various embodiments may be combined into one entity, which may perform functions identical to those of the relevant components before the combination.

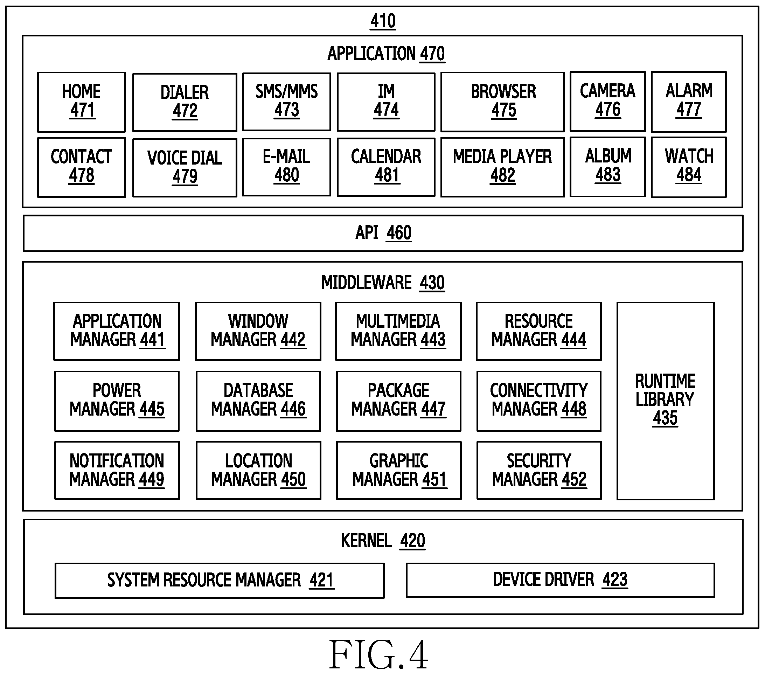

FIG. 4 is a diagram of a program module, according to an embodiment of the present disclosure. A program module 410 (e.g., the program 240) may include an operating system (OS) for controlling resources related to the electronic device (e.g., the electronic device 201) and/or various applications (e.g., the application programs 247) executed in the operating system. The operating system may be, for example, Android™, iOS™, Window™s, Symbian™, Tizen™, Badav™, or the like.

The program module 410 may include a kernel 420, middleware 430, an application programming interface (API) 460, and/or applications 470. At least some of the program module 410 may be preloaded on the electronic device, or may be downloaded from an external electronic device (e.g., the electronic device 202 or 204, or the server 206).

The kernel 420 may include, for example, a system resource manager 421 and/or a device driver 423. The system resource manager 421 may perform the control, allocation, retrieval, or the like of system resources. The system resource manager 421 may include a process manager, a memory manager, a file system manager, or the like. The device driver 423 may include, for example, a display driver, a camera driver, a Bluetooth driver, a shared memory driver, a USB driver, a keypad driver, a Wi-Fi driver, an audio driver, or an inter-process communication (IPC) driver.

The middleware 430 may provide, for example, a function commonly required by the applications 470, or may provide various functions to the applications 470 through the API 460 so that the applications 470 can efficiently use limited system resources within the electronic device. The middleware 430 (e.g., the middleware 243) may include, for example, at least one of a runtime library 435, an application manager 441, a window manager 442, a multimedia manager 443, a resource manager 444, a power manager 445, a database manager 446, a package manager 447, a connectivity manager 448, a notification manager 449, a location manager 450, a graphic manager 451, and a security manager 452.

The runtime library 435 may include a library module which a compiler uses in order to add a new function through a programming language while the applications 470 are being executed. The runtime library 435 may perform input/output management, memory management, the functionality for an arithmetic function, or the like.

The application manager 441 may manage, for example, a life cycle of at least one of the applications 470. The window manager 442 may manage Graphical User Interface (GUI) resources used for the screen. The multimedia manager 443 may determine a format required to reproduce various media files, and may encode or decode a media file by using a coder/decoder (codec) appropriate for the corresponding format. The resource manager 444 may manage resources, such as a source code, a memory, a storage space, and the like of at least one of the applications 470.

The power manager 445 may operate together with a basic input/output system (BIOS) to manage a battery or power, and may provide power information required for the operation of the electronic device. The power manager 445 may perform a control to charge or discharge the battery through at least one of a wired and wireless manner.

The database manager 446 may generate, search for, and/or change a database to be used by at least one of the applications 470. The package manager 447 may manage the installation or update of an application distributed in the form of a package file.

The connectivity manager 448 may manage a wireless connection such as, for example, Wi-Fi or Bluetooth. The notification manager 449 may display or notify of an event, such as an arrival message, an appointment, a proximity notification, and the like, in such a manner as not to disturb the user. The location manager 450 may manage location information of the electronic device. The graphic manager 451 may manage a graphic effect, which is to be provided to the user, or a user interface related to the graphic effect. The security manager 452 may provide various security functions required for system security, user authentication, and the like.

When the electronic device (e.g., the electronic device 201) has a telephone call function, the middleware 430 may further include a telephony manager for managing a voice call function or a video call function of the electronic device.

The middleware 430 may include a middleware module that forms a combination of various functions of the above-described elements. The middleware 430 may provide a module specialized for each type of OS in order to provide a differentiated function. Also, the middleware 430 may dynamically delete some of the existing elements, or may add new elements.

The API 460 (e.g., the API 245) is, for example, a set of API programming functions, and may be provided with a different configuration according to an OS. For example, in the case of Android or iOS, one API set may be provided for each platform. In the case of Tizen, two or more API sets may be provided for each platform.

The applications 470 (e.g., the application programs 247) may include, for example, one or more applications which can provide functions such as home 471, dialer 472, SMS/MMS 473, instant message (IM) 474, browser 475, camera 476, alarm 477, contacts 478, voice dial 479, email 480, calendar 481, media player 482, album 483, clock 484, health care (e.g., measure exercise quantity or blood sugar), or environment information (e.g., atmospheric pressure, humidity, or temperature information).

The applications 470 may include an information exchange application supporting information exchange between the electronic device (e.g., the electronic device 201) and an external electronic device (e.g., the electronic device 202 or 204). The information exchange application may include, for example, a notification relay application for transferring specific information to an external electronic device or a device management application for managing an external electronic device.

For example, the notification relay application may include a function of transferring, to the external electronic device, notification information generated from other applications of the electronic device 201 (e.g., an SMS/MMS application, an e-mail application, a health management application, or an environmental information application). Further, the notification relay application can, for example, receive notification information from the external electronic device and provide the received notification information to a user.

The device management application may manage (e.g., install, delete, or update), for example, at least one function of an external electronic device communicating with the electronic device (e.g., a function of turning on/off the external electronic device itself (or some components) or a function of adjusting luminance (or a resolution) of the display), applications operating in the external electronic device, or services provided by the external electronic device (e.g., a call service and a message service).

The applications 470 may include applications (e.g., a health care application of a mobile medical appliance or the like) designated according to attributes of the external electronic device 202 or 204. The application 470 may include an application received from the external electronic device. The application 470 may include a preloaded application or a third party application which can be downloaded from the server. Names of the elements of the program module 410, according to the above-described embodiments of the present disclosure, may change depending on the type of OS.

At least some of the program module 410 may be implemented in software, firmware, hardware, or a combination of two or more thereof. At least some of the program module 410 may be implemented (e.g., executed) by, for example, the processor (e.g., the processor 310). At least some of the program module 410 may include, for example, a module, a program, a routine, a set of instructions, and/or a process for performing one or more functions.

According to various embodiments of the present disclosure, an electronic device may include a communication module, a memory for storing multiple recognition algorithms for analyzing user activities, and a processor, wherein the processor may acquire activity schedule information related to the user activities; select one or more recognition algorithms among the multiple recognition algorithms, at least based on the activity schedule information; and analyze, using the one or more recognition algorithms, activity information related to the user activity and provide activity guide information related to the user activity based on the analyzed activity information.

According to various embodiments of the present disclosure, the electronic device may further include multiple sensors, wherein the processor may be configured to select one or more sensors among the multiple sensors, at least based on the one or more recognition algorithms, and acquire the activity information using the one or more sensors.

According to various embodiments of the present disclosure, the processor may be configured to deactivate one or more sensors that are not selected among the multiple sensors.

According to various embodiments of the present disclosure, the processor may be configured to acquire at least a part of the activity information of a user from an external device functionally connected with the electronic device.

According to various embodiments of the present disclosure, the processor may be configured to acquire the activity schedule information related to the user activities from an external device functionally connected with the electronic device.

According to various embodiments of the present disclosure, the processor may be configured to select a piece of activity schedule information from activity schedule information related to multiple user activities stored in the memory, based on the location of the electronic device, weather, time, or a combination thereof.

According to various embodiments of the present disclosure, the electronic device may further include a communication interface, wherein the processor may be configured to transmit activity guide information related to the user activities to an external device functionally connected with the electronic device, through the communication interface.

According to various embodiments of the present disclosure, the processor may be configured to analyze the activity information and update the activity schedule information.

The electronic device may identify multiple recognition algorithms for recognizing activity information of a user. For example, the electronic device may recognize activity information of a user using at least one of a zero-cross algorithm, an energy level algorithm, a vertical movement algorithm, a horizontal movement algorithm, and a gravity coordinates algorithm.

Each of the recognition algorithms may be designated for a different sensor for detecting activity information of a user like TABLE 1. For example, in TABLE 1 described below, a sensor having "0" displayed therefor may indicate a sensor for detecting activity information of a user in the corresponding recognition algorithm.

**TABLE 1**

| Algorithm | acceleration | gyro | geomagnetic | pressure |
|---|---|---|---|---|
| Zero Cross | O | O | | |
| Energy Level | O | | | |
| Vertical Movement | O | | | O |
| Horizontal Movement | O | | | O |
| Gravity Coordinates | O | O | O | O |

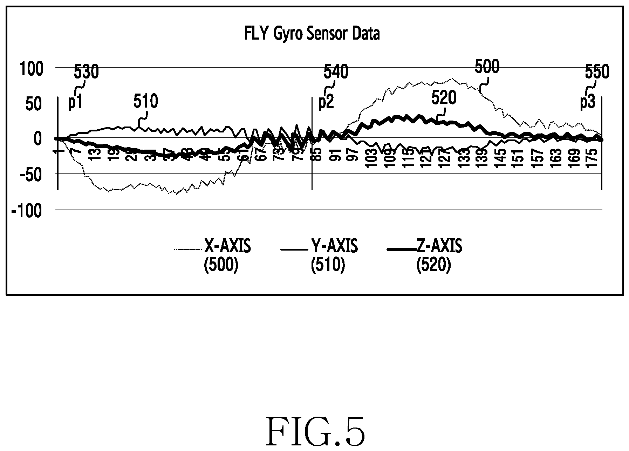

The zero-cross algorithm may indicate the recognition algorithm for acquiring activity information of a user by a rotational motion. For example, the electronic device may acquire activity information of a user according to a fly exercise, using sensing data like FIG. 5 according to the zero-cross algorithm.

FIG. 5 is a graph of a zero-cross algorithm, according to an embodiment of the present disclosure. In the description below, the horizontal axis of the graph may indicate time and the vertical axis of the graph may indicate the size of a signal.

Referring to FIG. 5, an electronic device may acquire, through a gyroscope, activity information of a user for a movement in which the user bends an arm (p2 540) from a state where the arm has been stretched out (p1 530), and stretch out the arm (p3 550) again, based on change information of x-axis 500 showing the range of change greater than other axis (e.g., y-axis 510) and z-axis 520.

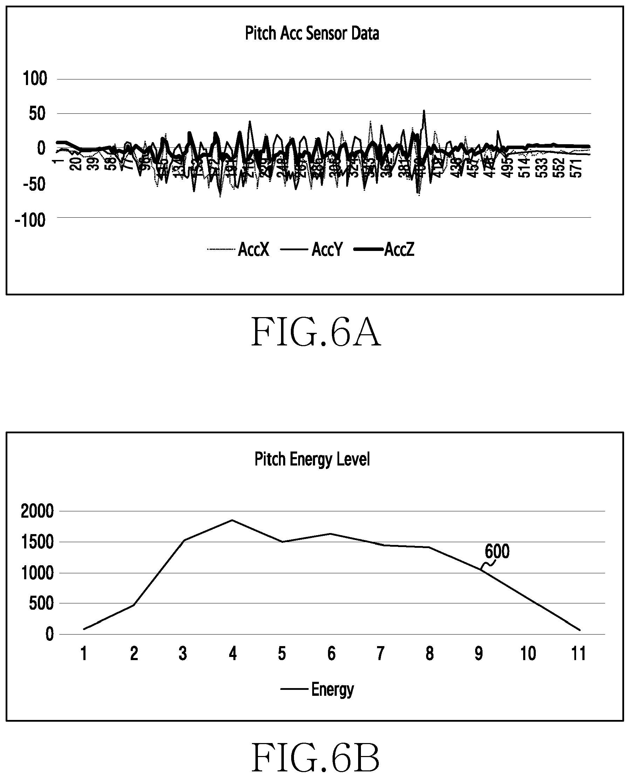

The energy level algorithm may indicate the recognition algorithm for acquiring activity information of a user, based on the size of acceleration generated by movements of the user. For example, the electronic device may acquire activity information of a user according to a stationary jump (pitch) exercise using sensing data like FIG. 6 according to the energy level algorithm.

FIGs. 6A and 6B are a graphs of an energy level algorithm, according to an embodiments of the present disclosure. In the description below, the horizontal axis of the graph may indicate time, and the vertical axis of the graph may indicate the size of a signal.

Referring to FIG. 6A, an electronic device may detect, through an acceleration sensor, raw data for each of an x-axis, y-axis, and z-axis according to a pitch exercise.

According to an embodiment, it is difficult to determine, using sensor data, the number of times of a pitch exercise performed by a user due to differences in the angle of arm movement or running posture for each user while performing the pitch exercise. Accordingly, the electronic device may acquire the quantity of movement (the size of acceleration) 600 according to the pitch exercise like FIG. 6 B, using raw data for each axis obtained through the acceleration sensor. For example, the electronic device may acquire an absolute value of movement of acceleration in which a gravitational factor is removed, as the activity information of a user according to the pitch exercise.

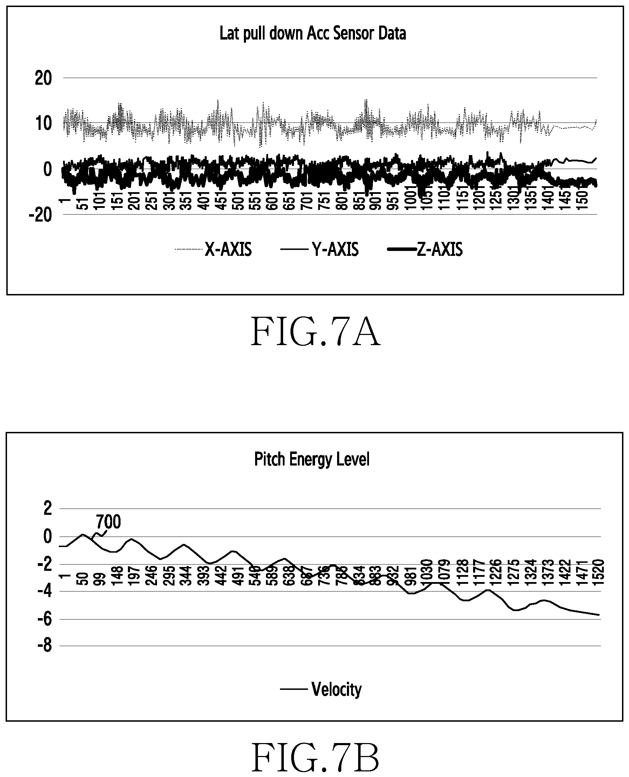

The vertical movement algorithm may indicate the recognition algorithm for recognizing a movement of a user with respect to the direction of gravity. For example, the electronic device may acquire activity information of a user according to a lat-pull-down exercise, using sensing data like FIG. 7 according to the vertical movement algorithm.

FIGs. 7A and 7B are graphs of a vertical movement algorithm, according to an embodiment of the present disclosure. In the description below, the horizontal axis of the graph may indicate time and the vertical axis of the graph may indicate the size of a signal.

Referring to FIG. 7A, an electronic device may detect, through an acceleration sensor, raw data for each of an x-axis, z-axis, and x-axis according to a lat-pull-down exercise.

The electronic device may convert raw data of each axis acquired through the acceleration sensor into velocity for the vertical direction like FIG. 7(b), so as to acquire activity information of a user (e.g., the number of times of exercise) according to a lat-pull-down exercise.

The horizontal movement algorithm may indicate the recognition algorithm for recognizing a movement of a user with respect to the direction towards ground. For example, the electronic device may convert raw data for each axis acquired through the acceleration sensor into velocity for the horizontal direction, so as to acquire activity information of a user (e.g., the number of times of exercise).

The gravity coordinates algorithm may indicate the recognition algorithm for correcting sensor data analyzed through at least one other algorithm. For example, the gravity coordinates algorithm may convert sensor data acquired through the acceleration sensor with reference to a world coordinate system, using a rotation vector. That is, the gravity coordinates algorithm may correct sensing data to be used for the vertical movement algorithm or the horizontal movement algorithm. For example, the gravity coordinates algorithm may provide additional information on movements of the electronic device on the world coordinate system by detecting, using a barometer, the vertical or horizontal movement of the electronic device. In this case, if a change generated in the barometer exceeds a reference value, the electronic device may determine that the change is not a horizontal directional exercise, so as to perform a control such that activity information of a user is not recognized, using the horizontal movement algorithm. Further, the electronic device may check whether a change in the barometer is generated, so as to improve the accuracy of activity information of a user, using the vertical movement algorithm.

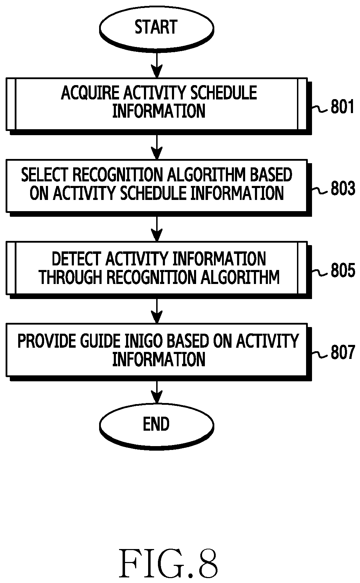

FIG. 8 is a flow-chart of a method for providing guide information based on activity information of a user by an electronic device, according to an embodiment of the present disclosure.

Referring to FIG. 8, in operation 801, an electronic device (e.g., the electronic device 201) may acquire activity schedule information (e.g., an exercise program). For example, the processor 220 may receive, through the communication interface 270, activity schedule information from the server 206 (e.g., a health care server, an exercise service providing server, or a schedule management server) or the other electronic device 202 or 204 (e.g., an electronic device of a trainer). For example, the processor 220 may extract activity schedule information stored in the memory 230. For example, the processor 220 may identify activity schedule information input through the input/output interface 250. For example, the activity schedule information may include, like TABLE 2 described below, exercise schedule information including the number of times of exercise, exercise type, exercise time, exercise sequence, exercise intensity, exercise place, number of repetitions, break time, biometric information (e.g., heart rate, oxygen saturation, etc.) according to the performance of exercise, and so on.

**TABLE 2**

| | WEEK 1 |
|---|---|
| DAY 1 | 30 minutes |
| | Warm Up |
| | - PT Jump 20 times |
| | - Jumping Jacks 30 seconds |
| | Repeat two sets (10 to 15 sec. break between exercises) Stretching |
| | 10 seconds per part (no break between exercises) Squat (using a gymball or a chair) - 12 times * including posture correction |
| | Wide squat 10 times |
| | Lunge (total 10 times considering both right foot and left foot lunge to be one time) |
| | Crunch 10 times |
| | Perform three sets (30 sec. break between sets) |
| DAY 2 | No exercise, perform only light aerobic exercise |
| | -jogging, walking, cycling (app. 30 minutes) |

In operation 803, the electronic device may select one or more recognition algorithms to be used for extracting activity information of a user, based on activity schedule information. For example, the memory 230 may store a table including at least one piece of recognition algorithm information corresponding to movement characteristics of the user for each exercise type. The processor 220 may select, in the memory 230, the recognition algorithm corresponding to an exercise type to be performed by the user, included in activity schedule information. For example, the processor 220 may extract the recognition algorithm corresponding to an exercise type to be performed by the user from activity schedule information configured in the form of a markup language.

In operation 805, the electronic device may detect activity information of a user through one or more recognition algorithms. For example, the processor 220 may activate one or more sensors configured in one or more recognition algorithms among multiple sensors included in the sensor 280. The processor 220 may acquire activity information of a user through the one or more activated sensors. For example, the processor 220 may select a peripheral device for acquiring activity information of a user, based on the one or more sensors configured in one or more recognition algorithms. The processor 220 may acquire activity information of a user through one or more peripheral devices.

In operation 807, the electronic device may provide exercise guide information based on the detected activity information of a user through one or more recognition algorithms. For example, the processor 220 may generate exercise guide information including information related to the current exercise state, information related to posture correction, etc., based on activity information of a user. For example, information related to an exercise state may include the number of times of exercise, exercise time, burned calories, biometric change information, the number of repetitions, and so on. For example, the processor 220 may control at least one of the speaker and display 260 of the input/output interface 250 to output exercise guide information. For example, the processor 220 may control the communication interface 270 to transmit exercise guide information to one or more peripheral devices, based on output characteristics of the exercise guide information.

The electronic device may output exercise guide information based on activity schedule information. For example, the electronic device may output exercise start information through an output module or a peripheral device, based on activity schedule information.

An electronic device that is worn on the wrist part of a user may store a table for exercise information capable of acquiring activity information of a user like TABLE 3 described below. For example, TABLE 3 described below may display the recognition algorithm to be used for acquiring activity information of a user for each of exercises as "0".

**TABLE 3**

| Sports events | Zero Cross | Energy Level | Vertical Mov. | Horizontal Mov. | Gravity Coordinates |
|---|---|---|---|---|---|
| Pitch | | O | | | |
| Rope Jump | O | | | | O |
| Burpee Test | O | | | | O |
| PT Jump | O | | | | |
| Jumping Jack | | O | | | |
| Gymball Squat | | | O | | O |
| Good Morning | O | | | | |
| Lunge | | | O | O | O |
| Squat | | | O | | O |
| Half Squat | | | O | | O |
| Back Squat | | | O | | O |
| Jump Squat | O | | | | O |
| Front Squat | | | O | | O |
| Chest Press | | | | O | O |
| Fly | O | | | | |
| Bench Press | | | O | | O |
| Crunch | O | | | | |
| Sit Up | O | | | | |
| Plank | | O | | | |
| Incline Bench Press | | | O | | O |
| Decline Bench Press | | | O | | O |
| Dumbbell Press | | | O | | O |
| Incline Dumbbell Press | | | O | | O |
| Decline Dumbbell Press | | | O | | O |
| Push Up | O | | | | |
| Lat Pull Down | | | O | | O |
| Cable Row | | | | O | O |
| Barbell Row | | | O | | O |
| Dead Lift | | | O | | O |
| Undergrip Lat Pull Down | | | O | | O |
| Barbell Good Morning | O | | | | |
| Barbell Shoulder Press | | | O | | O |
| Dumbbell Shoulder Press | | | O | | O |
| Dumbbell Front Raise | O | | | | |
| Dumbbell Lateral Raise | O | | | | |
| Dumbbell Bent Over Lateral Raise | O | | | | |
| Machine Shoulder Press | | | O | | O |
| EZ bar Curl | O | | | | |
| Dumbbell Curl | O | | | | |
| Hammer Curl | O | | | | |
| Concentration Curl | O | | | | |
| Cable Triceps Extension | O | | | | |
| Over Head Cable Triceps | O | | | | |
| Dumbbell Kick Back | O | | | | |
| Narrow Grip Bench Press | O | | | | |
| Lying Triceps Extension | O | | | | |
| One Arm Dumbbell Triceps Extension | O | | | | |
| Leg Curl | | | | | O |
| Leg Extension | | | | | O |
| Leg Press | | | | | O |
| Power Leg Press | | | | | O |
| Abduction | | | | | O |
| Adduction | | | | | O |
| Leg Raise | | | | | O |

An electronic device that is worn on the head part of a user may store a table for exercise information capable of acquiring activity information of a user like TABLE 4 described below. For example, TABLE 4 described below may display the recognition algorithm to be used for acquiring activity information of a user for each of exercises as "0".

**TABLE 4**

| Sports events | Zero Cross | Energy Level | Vertical Mov. | Horizontal Mov. | Gravity Coordinates |
|---|---|---|---|---|---|
| Pitch | | O | | | |
| Rope Jump | | | O | | O |
| Burpee Test | | | O | O | O |
| PT Jump | | | O | | |
| Jumping Jack | | O | | | |
| Gymball Squat | | | O | | O |
| Good Morning | O | | | | |
| Lunge | | | O | O | O |
| Squat | | | O | | O |
| Half Squat | | | O | | O |
| Back Squat | | | O | | O |
| Jump Squat | | | O | | O |
| Front Squat | | | O | | O |
| Chest Press | | | | O | O |
| Fly | | | | | O |
| Bench Press | | | | | O |
| Crunch | O | | | | |
| Sit Up | O | | | | |
| Plank | | O | | | O |
| Incline Bench Press | | | O | | O |
| Decline Bench Press | | | O | | O |
| Dumbbell Press | | | O | | O |
| Incline Dumbbell Press | | | O | | O |
| Decline Dumbbell Press | | | O | | O |
| Push Up | | | O | | O |
| Lat Pull Down | | | O | | O |
| Cable Row | | | | O | O |
| Barbell Row | | | O | | O |
| Dead Lift | | | O | | O |
| Undergrip Lat Pull Down | | | O | | O |
| Barbell Good Morning | O | | | | |
| Barbell Shoulder Press | | | O | | O |
| Dumbbell Shoulder Press | | | O | | O |
| Dumbbell Front Raise | | | | | O |
| Dumbbell Lateral Raise | | | | | O |
| Dumbbell Bent Over Lateral Raise | | | | | O |
| Machine Shoulder Press | | | | | O |
| EZ bar Curl | | | | | O |
| Dumbbell Curl | | | | | O |
| Hammer Curl | | | | | O |
| Concentration Curl | | | | | O |
| Cable Triceps Extension | | | | | O |
| Over Head Cable Triceps | | | | | O |
| Dumbbell Kick Back | | | | | O |
| Narrow Grip Bench Press | | | | | O |
| Lying Triceps Extension | | | | | O |
| One Arm Dumbbell Triceps Extension | | | | | O |
| Leg Curl | | | | | O |
| Leg Extension | | | | | O |
| Leg Press | | | | | O |
| Power Leg Press | | | | | O |
| Abduction | | | | | O |
| Adduction | | | | | O |
| Leg Raise | | | | | O |

An electronic device that is worn on the waist part of a user may store a table for exercise information capable of acquiring activity information of a user like TABLE 5 described below. For example, TABLE 5 described below may display the recognition algorithm to be used for acquiring activity information of a user for each of exercises as "0".

**TABLE 5**

| Sports events | Zero Cross | Energy Level | Vertical Mov. | Horizontal Mov. | Gravity Coordinates |
|---|---|---|---|---|---|
| Pitch | | ○ | | | |
| Rope Jump | | | ○ | | ○ |
| Burpee Test | | | ○ | ○ | ○ |
| PT Jump | | | ○ | | |
| Jumping Jack | | ○ | | | |
| Gymball Squat | | | ○ | | ○ |
| Good Morning | | | | | ○ |
| Lunge | | | ○ | ○ | ○ |
| Squat | | | ○ | | ○ |
| Half Squat | | | ○ | | ○ |
| Back Squat | | | ○ | | ○ |
| Jump Squat | | | ○ | | ○ |
| Front Squat | | | ○ | | ○ |
| Chest Press | | | | ○ | ○ |
| Fly | | | | | ○ |
| Bench Press | | | | | ○ |
| Crunch | ○ | | | | |
| Sit Up | ○ | | | | |
| Plank | | ○ | | | ○ |
| Incline Bench Press | | | | | ○ |
| Decline Bench Press | | | | | ○ |
| Dumbbell Press | | | | | ○ |
| Incline Dumbbell Press | | | | | ○ |
| Decline Dumbbell Press | | | | | ○ |
| Push Up | | | ○ | | ○ |
| Lat Pull Down | | | | | ○ |
| Cable Row | | | | | ○ |
| Barbell Row | | | | | ○ |
| Dead Lift | | | | | ○ |
| Undergrip Lat Pull Down | | | | | ○ |
| Barbell Good Morning | | | | | ○ |
| Barbell Shoulder Press | | | | | ○ |
| Dumbbell Shoulder Press | | | | | ○ |
| Dumbbell Front Raise | | | | | ○ |
| Dumbbell Lateral Raise | | | | | ○ |
| Dumbbell Bent Over Lateral Raise | | | | | ○ |
| Machine Shoulder Press | | | | | ○ |
| EZ bar Curl | | | | | ○ |
| Dumbbell Curl | | | | | ○ |
| Hammer Curl | | | | | ○ |
| Concentration Curl | | | | | ○ |
| Cable Triceps Extension | | | | | ○ |
| Over Head Cable Triceps | | | | | ○ |
| Dumbbell Kick Back | | | | | ○ |
| Narrow Grip Bench Press | | | | | ○ |
| Lying Triceps Extension | | | | | ○ |
| One Arm Dumbbell Triceps Extension | | | | | ○ |
| Leg Curl | | | | | ○ |
| Leg Extension | | | | | ○ |
| Leg Press | | | | | ○ |
| Power Leg Press | | | | | ○ |
| Abduction | | | | | ○ |
| Adduction | | | | | ○ |
| Leg Raise | | ○ | | | |

An electronic device that is worn on the leg part of a user may store a table for exercise information capable of acquiring activity information of a user like TABLE 6 described below. For example, TABLE6 described below may display the recognition algorithm to be used for acquiring activity information of a user for each of exercises as "0".

**TABLE 6**

| Sports events | Zero Cross | Energy Level | Vertical Mov. | Horizontal Mov. | Gravity Coordinates |
|---|---|---|---|---|---|
| Pitch | | ○ | | | |
| Rope Jump | | | ○ | | ○ |
| Burpee Test | | | ○ | ○ | ○ |
| PT Jump | | | ○ | | |
| Jumping Jack | | ○ | | | |
| Gymball Squat | | | | | ○ |
| Good Morning | | | | | ○ |
| Lunge | | | | | ○ |
| Squat | | | | | ○ |
| Half Squat | | | | | ○ |
| Back Squat | | | | | ○ |
| Jump Squat | | | | | ○ |
| Front Squat | | | | | ○ |
| Chest Press | | | | | ○ |
| Fly | | | | | ○ |
| Bench Press | | | | | ○ |
| Crunch | | | | | ○ |
| Sit Up | | | | | ○ |
| Plank | | ○ | | | ○ |
| Incline Bench Press | | | | | ○ |
| Decline Bench Press | | | | | ○ |
| Dumbbell Press | | | | | ○ |
| Incline Dumbbell Press | | | | | ○ |
| Decline Dumbbell Press | | | | | ○ |
| Push Up | | | | | ○ |
| Lat Pull Down | | | | | ○ |
| Cable Row | | | | | ○ |
| Barbell Row | | | | | ○ |
| Dead Lift | | | | | ○ |
| Undergrip Lat Pull Down | | | | | ○ |
| Barbell Good Morning | | | | | ○ |
| Barbell Shoulder Press | | | | | ○ |
| Dumbbell Shoulder Press | | | | | ○ |
| Dumbbell Front Raise | | | | | ○ |
| Dumbbell Lateral Raise | | | | | ○ |
| Dumbbell Bent Over Lateral Raise | | | | | ○ |
| Machine Shoulder Press | | | | | ○ |
| EZ bar Curl | | | | | ○ |
| Dumbbell Curl | | | | | ○ |
| Hammer Curl | | | | | ○ |
| Concentration Curl | | | | | ○ |
| Cable Triceps Extension | | | | | ○ |
| Over Head Cable Triceps | | | | | ○ |
| Dumbbell Kick Back | | | | | ○ |
| Narrow Grip Bench Press | | | | | ○ |
| Lying Triceps Extension | | | | | ○ |
| One Arm Dumbbell Triceps Extension | | | | | ○ |
| Leg Curl | ○ | | | | |
| Leg Extension | ○ | | | | |
| Leg Press | | | | ○ | ○ |
| Power Leg Press | | | | ○ | ○ |
| Abduction | ○ | | | | |
| Adduction | ○ | | | | |
| Leg Raise | ○ | | | | |

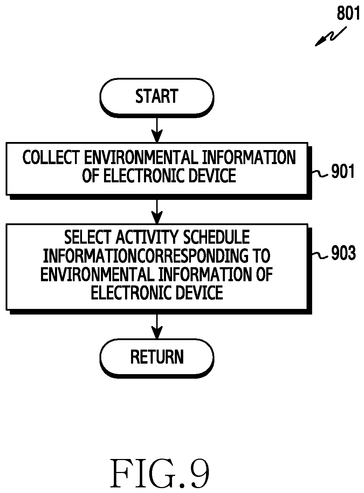

FIG. 9 is a flow-chart of a method for selecting activity schedule information corresponding to environmental information by an electronic device, according to an embodiment of the present disclosure. The description below explains an operation for acquiring activity schedule information in operation 801 of FIG. 8.

Referring to FIG. 9, in operation 901, an electronic device (e.g., the electronic device 201) may collect environmental information of the electronic device. For example, the processor 220 may collect environmental information such as a location of the electronic device 201, surrounding weather, and time.

In operation 903, the electronic device may select activity schedule information corresponding to environment information among multiple pieces of activity schedule information stored in the memory of the electronic device. For example, the processor 220 may select activity schedule information corresponding to a location of the electronic device 201 (e.g., a fitness club or a park) among multiple pieces of activity schedule information stored in the memory 230. For example, the processor 220 may select activity schedule information corresponding to a location of the electronic device 201 and surrounding weather among multiple pieces of activity schedule information stored in the memory 230.

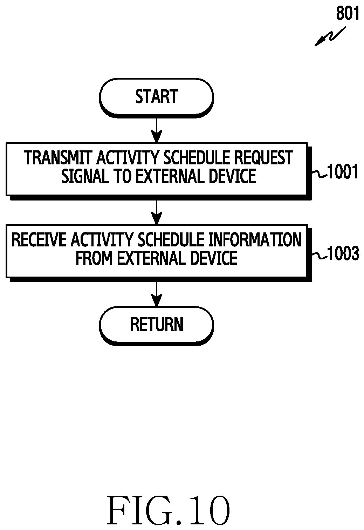

FIG. 10 is a flow-chart of a method for receiving, from an external device, activity schedule information by an electronic device, according to an embodiment of the present disclosure. The description below may explain an operation for acquiring activity schedule information in operation 801 of FIG. 8.

Referring to FIG. 10, in operation 1001, an electronic device (e.g., the electronic device 201) may transmit an activity schedule request signal to an external device (e.g., the first external electronic device 202, the second external electronic device 204, or the server 206) in response to generation of an activity schedule check event. For example, the processor 220 may control the communication interface 270 to transmit an activity schedule request signal to the external device when an activity schedule check period arrives. For example, the processor 220 may control the communication interface 270 to transmit an activity schedule request signal to the external device when an exercise service application is operated. For example, the processor 220 may control the communication interface 270 to transmit an activity schedule request signal to the external device when an input for an activity schedule check is detected. For example, the input for an activity schedule check may include at least one of input information detected through the input/output interface 250 and movement data, detected through the sensor 280, of the electronic device 201. For example, the activity schedule request signal may include environmental information of the electronic device 201.

In operation 1003, the electronic device may receive activity schedule information from an external device. For example, the processor 220 may receive, from an external device, activity schedule information corresponding to environmental information of the electronic device 201 through the communication interface 270. For example, the external device may include a server connected to communicate with the electronic device 201 for providing an exercise service or other electronic devices.

The electronic device may receive activity schedule information periodically transmitted by the server connected to communicate with the electronic device. For example, the processor 220 may receive activity schedule information transmitted by the server through the communication interface 270 when activity schedule check period arrives.

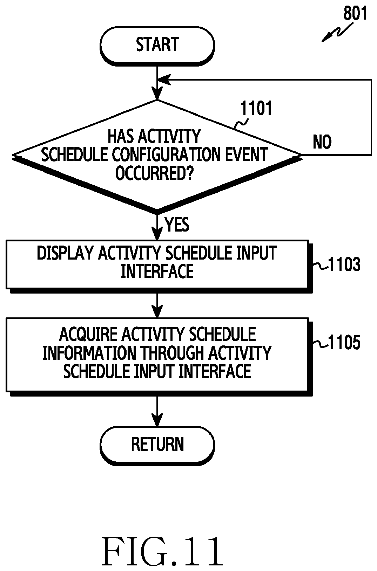

FIG. 11 is a flow-chart of a method for acquiring activity schedule information by an electronic device according to various embodiments of the present disclosure. The description below explains an operation for acquiring activity schedule information in operation 801 of FIG. 8.

Referring to FIG. 11, in operation 1101, an electronic device (e.g., the electronic device 201) may check whether an activity schedule configuration event occurs. For example, the processor 220 may check whether an input corresponding to the activity schedule configuration event is detected through the input/output interface 250. For example, the processor 220 may check, through the sensor 280, whether movements of the electronic device 201 or a gesture input corresponding to the activity schedule configuration event is detected. For example, the processor 220 may check whether an exercise service application is operated. For example, the processor 220 may determine that the activity schedule configuration event has occurred when the exercise service application is operated.

In operation 1103, the electronic device may display an activity schedule input interface in at least a part of a display when an occurrence of an activity schedule configuration event is detected. For example, the processor 220 may control the display 260 to display a pop-up window for an activity schedule input in response to the occurrence of the activity schedule configuration event.

In operation 1105, the electronic device may acquire activity schedule information based on input information corresponding to the activity schedule configuration input interface displayed on the display. For example, the processor 220 may generate an exercise program based on information input through the activity schedule input interface displayed on the display 260. An exercise program according to various embodiments may include exercise schedule information including the number of times of exercise, exercise type, exercise time, exercise sequence, exercise intensity, the number of repetitions, and so on.

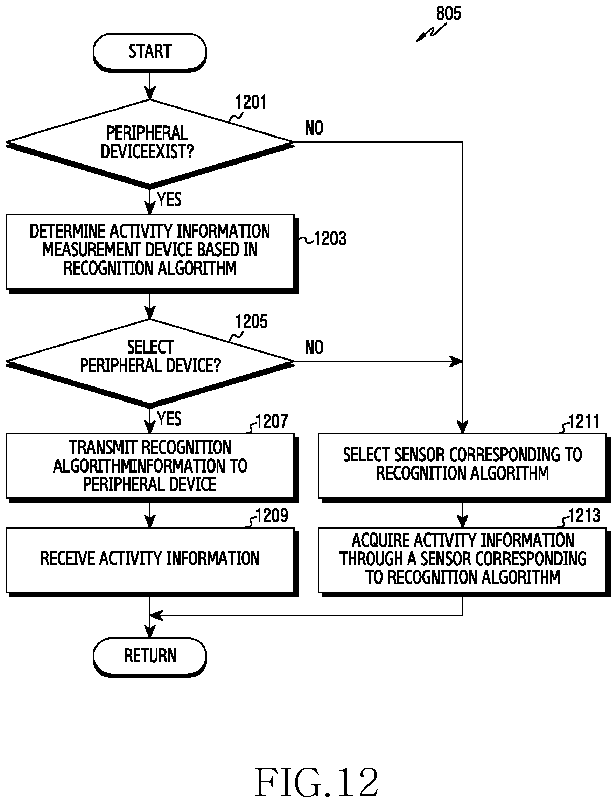

FIG. 12 is a flow-chart of a method for acquiring activity information on a user by an electronic device, according to an embodiment of the present disclosure. The description below may explain an operation for detecting activity information of a user in operation 805 of FIG. 8.

Referring to FIG. 12, in operation 1201, an electronic device (e.g., the electronic device 201) may check whether a peripheral device (e.g., the first external electronic device 202 or the second external electronic device 204) exists. For example, the processor 220 may check whether a peripheral device connected to communicate with the electronic device 201 exists. For example, the processor 220 may check whether a peripheral device capable of being connected to communicate with the electronic device 201 exists by performing a scan operation. For example, the peripheral device may include the smart headset 112, the smart band 114, the smart shoe 118, and smart sporting goods including one or more sensors, illustrated in FIG. 1.

In operation 1203, the electronic device may determine a measurement device for measuring activity information of a user, based on one or more recognition algorithms selected to extract activity information of the user when one or more peripheral devices exists. For example, the processor 220 may compare one or more sensors configured in one or more recognition algorithms with a sensor list of peripheral devices, so as to select a peripheral device for acquiring activity information of the user. For example, the processor 220 may select a peripheral device for acquiring activity information of the user, based on one or more recognition algorithms and a list of recognition algorithms capable of supporting the peripheral device. For example, the processor 220 may identify peripheral device information mapped to the recognition algorithm included in activity schedule information. The processor 220 may select a peripheral device mapped to the recognition algorithm among one or more peripheral devices. For example, the processor 220 may determine the measurement device corresponding to a particular exercise, based on the priority and remaining battery of each of devices when multiple devices capable of measuring activity information of the user corresponding to the particular exercise exist.

In operation 1205, the electronic device may check whether a peripheral device is selected as a measurement device for measuring activity information of a user.

In operation 1207, the electronic device may transmit information on at least recognition algorithm to a peripheral device selected as a measurement device when the peripheral device is selected as the measurement device for measuring activity information of a user. For example, the processor 220 may control the communication interface 270 to transmit, to a corresponding peripheral device, one or more recognition algorithms corresponding the peripheral device selected as the measurement device. For example, the processor 220 may control the communication interface 270 to transmit, to a corresponding peripheral device, sensor information configured in the recognition algorithm corresponding to the peripheral device selected as the measurement device.

In operation 1209, the electronic device may receive activity information of a user from a peripheral device selected as a measurement device. For example, the processor 220 may receive, from a corresponding peripheral device, activity information of a user determined by the peripheral device through the communication interface 270. For example, the peripheral device may transmit, to the electronic device 201, activity information of a user detected by analyzing sensor data (e.g., raw data) collected through one or more sensors corresponding to one or more recognition algorithms. For example, the processor 220 may receive, from a corresponding peripheral device, sensor data collected by the peripheral device using the sensor, through the communication interface 270. The processor 220 may detect activity information of the user by analyzing sensor data provided by the peripheral device.

In operation 1211, the electronic device may select one or more sensors corresponding to one or more recognition algorithms when a peripheral device does not exist or a peripheral device is not selected as a measurement device for measuring activity information of a user. For example, the processor 220 may select one or more sensors corresponding to one or more recognition algorithms among multiple sensors included in the sensor 280. For example, the processor 220 may deactivate a sensor that is not selected.

In operation 1213, the electronic device may detect activity information of a user (e.g., body movements or biometric information) through one or more sensors corresponding to one or more recognition algorithms. For example, the electronic device may detect activity information of a user by analyzing sensor data collected through one or more sensors corresponding to one or more recognition algorithms.

According to an embodiment, the electronic device 201 may select one or more peripheral devices and the electronic device 201 as measurement devices for measuring activity information of a user, in operation 1203. In this case, the electronic device 201 may perform, in parallel, an operation (operations 1207 and 1209) for receiving activity information from the peripheral device and an operation (operations 1211 and 1213) for detecting activity information of a user through one or more sensors.

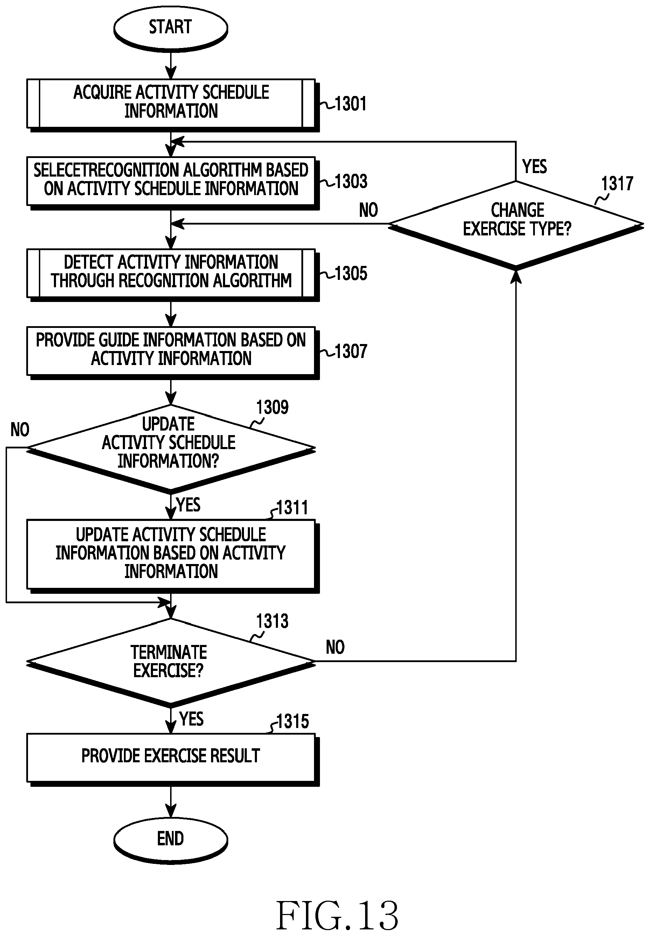

FIG. 13 is a flow-chart of a method for updating activity schedule information by an electronic device, according to an embodiment of the present disclosure.

Referring to FIG. 13, in operation 1301, an electronic device may acquire activity schedule information (e.g., an exercise program or an exercise schedule) for providing an exercise service. For example, the processor 220 may extract activity schedule information stored in the memory 230 like FIG. 9. For example, the processor 220 may receive activity schedule information from an external device like FIG. 10. For example, the processor 220 may generate activity schedule information based on input information like FIG. 11. For example, the processor 220 may generate activity schedule information or update predetermined activity schedule information, based on activity information of a user acquired through the sensor 280. For example, activity schedule information may include exercise program information including the number of times of exercise, exercise type, exercise time, exercise sequence, exercise intensity, exercise place, the number of repetitions, and so on.

In operation 1303, the electronic device may select one or more recognition algorithms to be used for detecting activity information of a user corresponding to activity schedule information among predefined multiple recognition algorithms for recognizing activity information of the user. For example, the processor 220 may check an exercise type to be performed by the user, based on activity schedule information. The processor 220 may select one or more recognition algorithms corresponding to the exercise type to be performed by the user, with reference to TABLE 4 to TABLE 6.

In operation 1305, the electronic device may detect activity information of a user through one or more recognition algorithms. For example, the processor 220 may detect activity information of a user with respect to an exercise performed based on activity schedule information, using at least one of a peripheral device and the electronic device 201 like FIG. 12.

In operation 1307, the electronic device may provide exercise guide information based on activity information of a user. For example, the processor 220 may detect an exercise quantity of a user with respect to a particular exercise scheme, based on activity information of the user. The processor 220 may output exercise quantity information of the user through the speaker or display 260 of the input/output interface 250. For example, the processor 220 may transmit exercise quantity information of the user to one or more peripheral devices. For example, the processor 220 may perform a control to output a beep sound through the speaker or smart headset 112 of the input/output interface 250 each time when jumping rope is performed five times. For example, the processor 220 may perform a control to output posture correction information for a crunch exercise to the display 260 or the smart glasses 116.

In operation 1309, the electronic device may determine whether to update activity schedule information, based on activity information (e.g., biometric information) of a user. For example, the processor 220 may determine whether to update activity schedule information, based on the user's heart rate or oxygen saturation during an exercise. For example, the processor 220 may determine whether to update activity schedule information, based on the user's heart rate or oxygen saturation in a break time period. For example, the processor 220 may determine to update activity schedule information when the user's heart rate exceeds a reference value. For example, the processor 220 may determine whether to update activity schedule information, based on activity information of another user provided by an external device. For example, the processor 220 may determine to update activity schedule information when the types of exercise to be performed by the user of the electronic device 201 and another user are the same.

In operation 1313, the electronic device may check whether an exercise is terminated when it is determined not to update activity schedule information.

In operation 1311, the electronic device may update activity schedule information to correspond to activity information of a user when it is determined not to update the activity schedule information. For example, the processor 220 may lower exercise intensity, change the exercise sequence, or increase a break period when the user's heart rate increases up to a reference heart rate or above, or oxygen saturation goes down to a reference value or below. For example, the processor 220 may change the exercise sequence or change a break period when the types of exercise to be performed by the user of the electronic device 201 and another user are the same. For example, the processor 220 may change the exercise sequence of the user to be same as or different from the exercise sequence of another user.

In operation 1313, the electronic device may check whether a user's exercise is terminated. For example, the processor 220 may check whether an operation of an exercise application is terminated, based on input information detected through the input/output interface 250. For example, the processor 220 may check whether an exercise schedule of the user is terminated, based on activity schedule information.

In operation 1317, the electronic device may check whether the type of exercise to be performed by a user is changed, based on activity schedule information when the exercise of the user has not been terminated.

In operation 1303, the electronic device may select one or more recognition algorithms corresponding to the type of exercise to be performed by a user when the type of exercise to be performed by the user has been changed.

In operation 1305, the electronic device may detect activity information of a user through one or more recognition algorithms when the type of exercise to be performed by the user has not been changed.

In operation 1315, the electronic device may provide the result of exercise corresponding to activity information of a user when the exercise of the user has been terminated. For example, the processor 220 may determine an achievement rate of exercise by comparing activity information of a user with activity schedule information. The processor 220 may control the display 260 to display the achievement rate. For example, the processor 220 may analyze activity information of the user and detect average biometric information of the user during an exercise. The processor 220 may control the display 260 to display the user's biometric information according to an exercise performance. For example, the user's biometric information may include at least one of a heart rate, oxygen saturation, and burned calories.

An electronic device (e.g., the electronic device 201) may display, on the display 260, the result of exercise and information related to the result of exercise received from an external device (e.g., electronic device 202 or 204) or the server 206. For example, the processor 220 may perform a control to display, on the display 260 through various methods, information on the result of previous exercise (e.g., records of exercise) of a user of the electronic device 201, which is received from the server 206 in addition to information on the result of exercise, in operation 1315 of FIG. 13. For example, the processor 220 may perform a control to display, on the display 260 through various methods, information on the result of exercise for a user of another electronic device received from the another electronic device (e.g., the electronic device 204) in addition to information on the result of exercise for the user of the electronic device 201, in operation 1315 of FIG. 13.

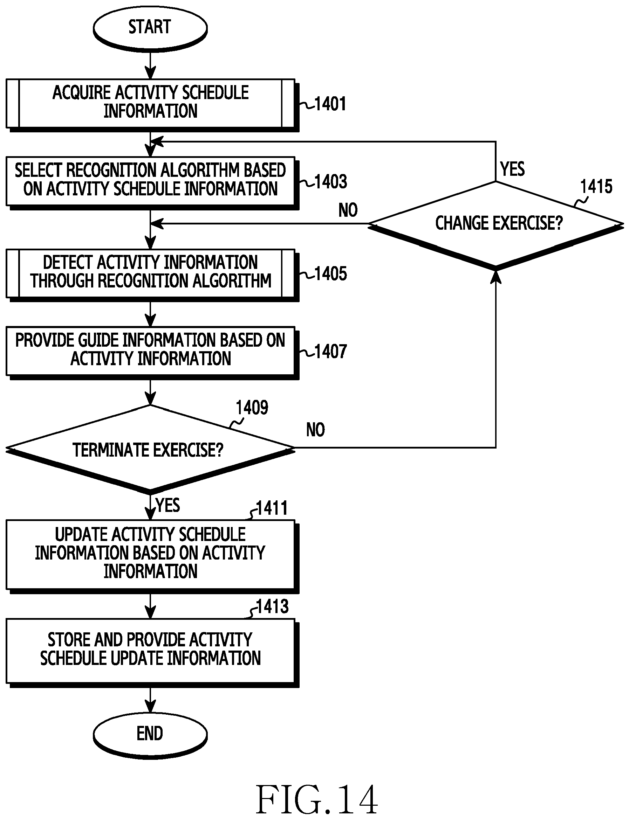

FIG. 14 is a flow-chart of a method for updating activity schedule information by an electronic device, according to an embodiment of the present disclosure.

Referring to FIG. 14, in operation 1401, an electronic device 201 may acquire activity schedule information (e.g., an exercise program, activity schedule information, or the like) for providing an exercise service. For example, the processor 220 may extract activity schedule information stored in the memory 230 like FIG. 9. For example, the processor 220 may receive activity schedule information form an external device like FIG. 10. For example, the processor 220 may generate activity schedule information, based on input information like FIG. 11. For example, the processor 220 may generate activity schedule information or update predetermined activity schedule information, based on profile information of a user. For example, the profile information of a user may include the user's body size, age, blood pressure, and so on.

In operation 1403, the electronic device may select one or more recognition algorithms corresponding to activity schedule information among multiple recognition algorithms usable for recognizing activity information of a user. For example, the processor 220 may select one or more recognition algorithms corresponding to the type of exercise to be performed by a user among multiple recognition algorithms with reference to TABLE 4 to TABLE 6. For example, the processor 220 may identify the type of exercise to be performed by the user, in activity schedule information.

In operation 1405, the electronic device may detect activity information of a user through one or more recognition algorithms. For example, the processor 220 may detect activity information of a user for an exercise performed by the user, using at least one of a peripheral device and the electronic device 201 like FIG. 12.

In operation 1407, the electronic device may provide exercise guide information corresponding to activity information of a user. For example, the processor 220 may perform a control to output exercise guide information corresponding to activity information of a user through at least one of an output module of the electronic device 201 or a peripheral device. For example, the exercise guide information may include at least one of an exercise quantity of a user, posture correction information, and biometric information of a user, which are detected based on activity information of the user.

In operation 1409, the electronic device may check whether a user's exercise is terminated. For example, the processor 220 may check an operation of an exercise application is terminated, based on input information detected through the input/output interface 250. For example, the processor 220 may check whether the user's exercise schedule is terminated, based on activity schedule information.

In operation 1415, the electronic device may check whether the type of exercise to be performed by a user is changed, based on activity schedule information and exercise quantity of the user when the user's exercise has not been terminated. For example, the processor 220 may determine whether to continuously perform a first exercise scheme by comparing a target exercise quantity for the first exercise scheme configured in activity schedule information with the user's exercise quantity. For example, the processor 220 may determine to continuously perform the first exercise scheme when the user's exercise quantity is less than the target exercise quantity.

In operation 1403, the electronic device may select one or more recognition algorithms corresponding to the type of exercise (e.g., a second exercise scheme) to be performed by a user when the type of exercise to be performed by the user has been changed.

In operation 1405, the electronic device may detect activity information of a user through one or more recognition algorithms when the type of exercise to be performed by the user has not been changed.

In operation 1411, the electronic device may update activity schedule information to correspond to activity information of a user when the user's exercise has been terminated. For example, the processor 220 may change at least one of exercise intensity, exercise sequence, and a break period which are included in activity schedule information, based on changes in the user's biometric information (e.g., heart rate and oxygen saturation) measured during the exercise. That is, the processor 220 may update activity schedule information to be performed by the user tomorrow, based on activity information of the user performed today.

In operation 1413, the electronic device may store and provide updated activity schedule information. For example, the processor 220 may store updated activity schedule information on the memory 230. For example, the processor 220 may control the communication interface 270 to transmit updated activity schedule information to the server 206. For example, the processor 220 may control the display 260 to display updated activity schedule information. For example, the processor 220 may control the communication interface 270 to transmit updated activity schedule information to one or more peripheral devices including a display.

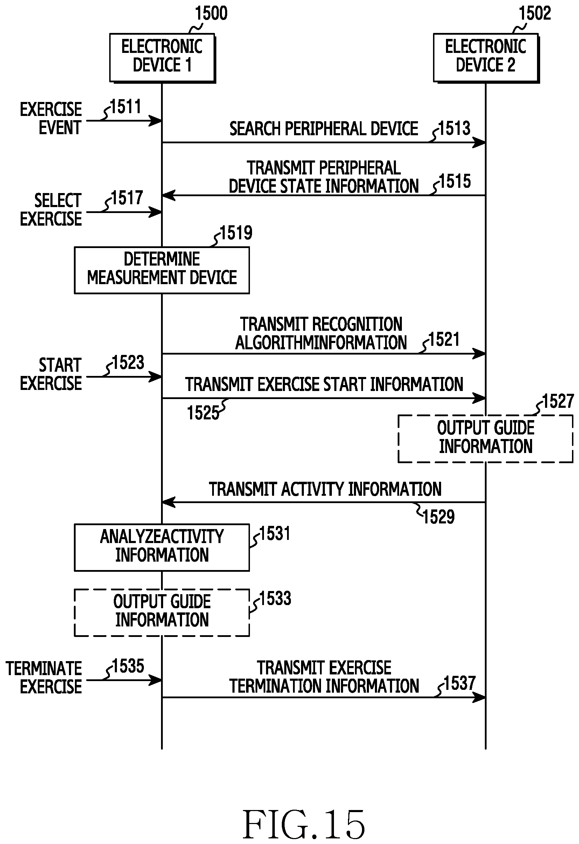

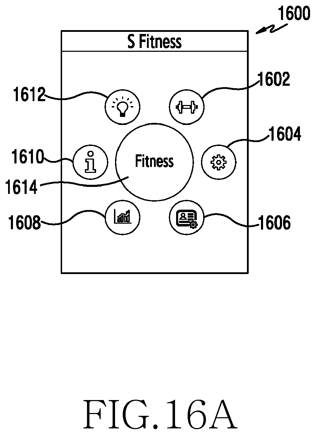

FIG. 15 is a signal flow diagram of a method for collecting activity information on a user, according to an embodiment of the present disclosure. The description below explains an operation for collecting activity information of a user, using a screen configuration illustrated in FIG. 16A-FIG. 16H. A first electronic device 1500 may include the smart watch 100 of FIG. 1, and a second electronic device 1502 may include the smart headset 112 of FIG. 1. In the description below, an operation displayed as a dotted line may be omitted.

Referring to FIG. 15, the first electronic device 1500 may perform 1513 a scan operation to check whether a peripheral device exists when an exercise event has occurred 1511. For example, the first electronic device 1500 may transmit a peripheral device search signal, using a short range communication scheme when an exercise application is operated, based on input information. For example, the first electronic device 1500 may display, on a display, an operation screen 1600 of an exercise application like FIG. 16a when the exercise application is operated. The operation screen 1600 of the exercise application may include one or more menu icons 1602 to 1612 and an execution icon 1614. The first electronic device 1500 may provide a service corresponding to a selected menu icon when selection of the execution icon 1614 is detected after selecting the menu icon. Here, the menu icon 1602 to 1612 may include an exercise execution icon 1602, a configuration icon for configuring an exercise application 1604, a profile icon including profile information of a user 1606, an exercise recording icon 1608, an information icon including an exercise application 1610, and a guide icon including exercise relating guide information 1612.

The second electronic device 1502 may transmit 1515 state information of the second electronic device 1502 to the first electronic device 1500 in response to the scan operation of the first electronic device 1500. For example, the second electronic device 1502 may transmit, to the first electronic device 1500, a sensor list and battery information of the second electronic device 1502 when the peripheral device search signal has been received. The sensor list of the second electronic device 1502 may include information on one or more sensors electrically connected to the second electronic device 1502. For example, the second electronic device 1502 may transmit, to the first electronic device 1500, a list of recognition algorithms and battery information of the second electronic device 1502 when a peripheral device search signal has been received. For example, the list of recognition algorithms of the second electronic device 1502 may include information on recognition algorithms applicable to the second electronic device 1502.

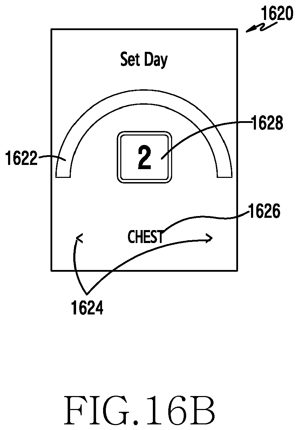

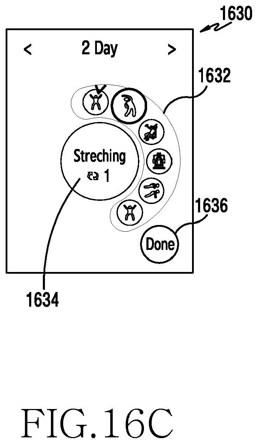

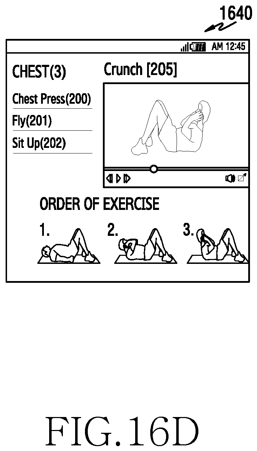

The first electronic device 1500 may determine that the second electronic device 1502 for interworking an exercise service is adjacent when state information of the second electronic device 1502 has been received. The first electronic device 1500 may determine 1519 a device to measure activity information of a user for a corresponding exercise when the exercise performed by the user is selected 1517. For example, the first electronic device 1500 may display an activity schedule screen 1620 on a display like FIG. 16B when the exercise execution icon 1602 is executed in FIG. 16A. The first electronic device 1500 may display exercise schedule information 1630 on a display like FIG. 16c when an input for selecting date information 1628 for performing exercise has been detected on the activity schedule screen 1620. The first electronic device 1500 may determine a device to measure activity information of the user for an exercise selected in an exercise list 1632 when an input for selecting detailed exercise information 1634 in exercise schedule information 1630 of FIG. 16C. In addition, the first electronic device 1500 may display, on a display, guide information 1640 on the exercise selected in the exercise list 1632 like FIG. 16D. For example, the activity schedule screen 1620 of FIG. 16B may include the current exercise state information 1622 and a date change icon 1624, exercise summary information 1626 of the corresponding date and date information 1628 for performing exercise, with respect to an exercise schedule included in activity schedule information. For example, exercise schedule information 1630 of FIG. 16C may include an exercise list 1632, detailed exercise information 1634, and a forced shutdown button 1636, corresponding to a date for performing exercise (e.g., day 2). The exercise list 1632 may display each of exercise icons to correspond to the exercise sequence performed by the user. An exercise icon selected by the user in the exercise list 1632 or recognized as being performed by the user may have a display parameter (e.g., color, size, transparency, etc.) that is modified to be distinguished from other exercise icons. An exercise icon, the exercise of which indicates has been performed by the user, may have a display parameter (e.g., color, size, transparency, etc.) that is modified to be distinguished from other exercise icons. The detailed exercise information 1634 may include detailed information on an exercise selected by the user or recognized as being performed by the user and the current exercise quantity information.

The first electronic device 1500 may transmit 1521, to the second electronic device 1502, recognition algorithm information corresponding to the second electronic device 1502, based on determination information of a measurement device. For example, the recognition algorithm information may include a list of recognition algorithms or a sensor list to be used by the second electronic device 1502 for detecting activity information of the user.

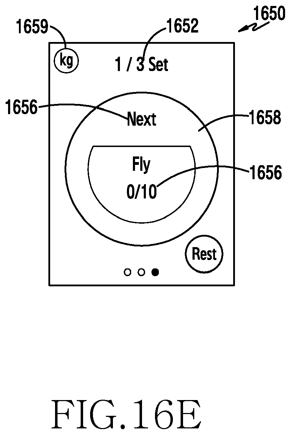

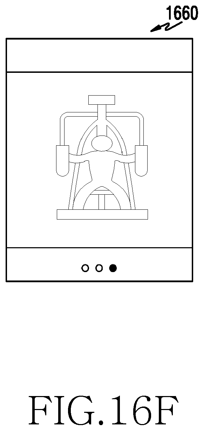

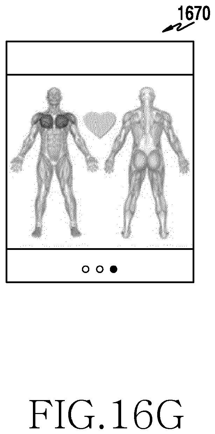

The first electronic device 1500 may transmit exercise start information to the second electronic device 1502 when input information to start exercise is detected 1523. For example, the first electronic device 1500 may display, on a display, an exercise performance screen 1650 like FIG. 16E when an input for selecting detailed exercise information 1634 in exercise schedule information 1630 of FIG. 16C is detected. The first electronic device 1500 may detect a time point to start exercise, based on selection information of information on the number of times of exercise 1656 displayed on the exercise performance screen 1650. For example, the exercise performance screen 1650 may include information on the number of repetitions of exercise 1652, an exercise type change icon 1654, information on the number of times of exercise 1656, and exercise achievement rate information 1658. The information on the number of times of exercise 1656 may further include information on a target time for performing the number of times of exercise. It may be determined to start or stop exercising based on selection of information on the number of times of exercise 1656. In addition, the first electronic device 1500 may display posture information 1660 of a corresponding exercise like FIG. 16F or information on a strengthening part 1670 like FIG. 16G when a right or left drag input on the exercise performance screen 1650 is detected.

The second electronic device 1502 may output 1527 exercise guide information, based on exercise start information received by the first electronic device 1500. For example, the second electronic device 1502 may output, through a speaker, a sound guidance to start exercise.

The second electronic device 1502 may collect activity information of a user according to exercise performance and transmit 1529 the activity information of a user to the first electronic device 1500 in response to the exercise start information received by the first electronic device 1500.

The first electronic device 1500 may analyze 1631 the activity information of a user provided by the second electronic device 1502. For example, the first electronic device 1500 may analyze the activity information of a user provided by the second electronic device 1502 and the activity information of a user measured by the first electronic device 1500, and generate exercise guide information. For example, the exercise guide information may include at least one of an exercise quantity and biometric information of a user.

The first electronic device 1500 may output 1533 exercise guide information generated based on activity information of a user. For example, the first electronic device 1500 may display an exercise quantity and biometric information of a user through the exercise performance screen 1650 like FIG. 16E.

The first electronic device 1500 may transmit exercise termination information to the second electronic device 1502 when an occurrence of an exercise termination event has been detected 1535. The second electronic device 1502 may stop collecting activity information of a user in response to the exercise termination information.

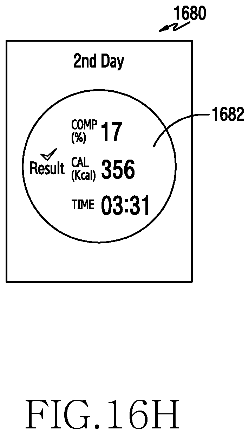

The first electronic device 1500 may display, on a display, information on the result of exercise 1680 like FIG. 16H when the exercise has been terminated. For example, the information on the result of exercise 1680 may include an exercise achievement rate and biometric information of a user according to exercise performance. In addition, the first electronic device 1500 may display, on a display, information on the result of exercise 1680 up to a time point when an input of the forced shutdown button 1636 has been detected like FIG. 16H when the input of the forced shutdown button 1636 of FIG. 16C has been detected.

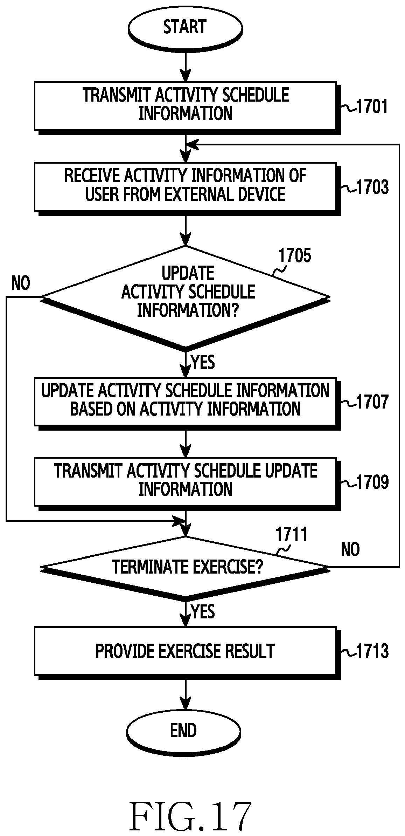

FIG. 17 is a flow-chart of a method for controlling an exercise service of an external device by an electronic device, according to an embodiment of the present disclosure. The description below explains an operation for controlling an exercise service of the smart watch 100 by the smart phone 110 of FIG. 1. The smart phone 110 of FIG. 1 may include all or some of the electronic device 201 of FIG. 2.

Referring to FIG. 17, in operation 1701, an electronic device may transmit, to an external device (e.g., the smart watch 100), activity schedule information (e.g., an exercise program) to be performed by a user of the external device. For example, the processor 220 may control the communication interface 270 to transmit activity schedule information to the external device through a short range communication scheme. For example, the processor 220 may control the communication interface 270 to transmit, to a server (e.g., the server 206) for an exercise service, activity schedule information to be transmitted to the external device. An electronic device (e.g., the electronic device 201) may transmit, to an external device, activity schedule information acquired from the server 206 (e.g., a health care management server and an exercise service providing server). For example, the electronic device may store activity schedule information acquired from the server 206 in the memory 230.

In operation 1703, the electronic device may receive, from an external device, activity information of a user corresponding to activity schedule information. For example, the processor 220 may periodically receive, from an external device, activity information of a user through the communication interface 270.

In operation 1705, the electronic device may determine whether to update activity schedule information of an external device, based on activity information of a user provided by the external device. For example, the processor 220 may determine whether to update activity schedule information of an external device, based on biometric information (e.g., heart rate or oxygen saturation) of the user provided by the external device.

In operation 1711, the electronic device may check whether the exercise of the external device is terminated when it is determined not to update activity schedule information of the external device.

In operation 1707, the electronic device may update activity schedule information of an external device to correspond to activity information of a user provided by the external device when it is determined not to update activity schedule information of the external device. For example, the processor 220 may update activity schedule information of the external device to correspond to a heart rate or oxygen saturation of the user provided by the external device. For example, the processor 220 may lower exercise intensity included in activity schedule information of the external device. The processor 220 may omit at least a part of exercise from an exercise list of activity schedule information of the external device. The processor 220 may change the exercise sequence included in activity schedule information of the external device. The processor 220 may increase a break period included activity schedule information of the external device.

In operation 1709, the electronic device may transmit, to an external device, updated activity schedule information. For example, the processor 220 may transmit activity schedule information to an external device or a server (e.g., the server 206) for an exercise service through the communication interface 270.

In operation 1711, the electronic device may check whether an exercise of an external device is terminated. For example, the processor 220 may check whether an exercise termination signal is received from an external device through the communication interface 270. For example, the processor 220 may check whether an exercise schedule for a user of an external device is terminated, based on activity schedule information to be transmitted to the external device.

In operation 1703, the electronic device may receive, from an external device, activity information of a user corresponding to activity schedule information when an exercise of the user has not been terminated.

In operation 1713, the electronic device may provide the result of exercise corresponding to activity information of a user provided by an external device when an exercise of the external device has been terminated. For example, the processor 220 may detect an exercise achievement rate of a user of an external device by comparing activity information of the user provided by the external device with activity schedule information. The processor 220 may display the exercise achievement rate on the display 260 or transmit the same to the external device. For example, the processor 220 may generate or update next activity schedule information, based on activity schedule information of a user provided by an external device. The processor 220 may store the next activity schedule information in the memory 230 or transmit the same to the external device.

An electronic device may transmit, to an external device, activity schedule information configured in the form of a markup language (e.g., XML) like TABLE7 described below. For example, activity schedule information may include exercise schedule information such as an exercise list, exercise intensity, the number of times of exercise, exercise sequence, like TABLE7 described below.

**TABLE 7**

| |
|---|
| ```
         Fitness program information
 <?xml version="1.0" encoding="utf-8"?>
 <fitnessprogram>
   <program id="0" name="Diet in Fitness Center" owner="0" creator="A"/>
     <duration year="2015" month="2" day="2" numberofday="20" />
     <day id="0" numberofgroup="5" currentgroup="0" date="2015-05-04" detail="
 Lower / circuit / chest ">
         <group id="0" repeat="2" middlerest="0" afterrest="0" mode="auto"
 numberofexercise="2" order="fixed">
            <exercise id="9" name="PT Jump" action="countable" targetcount="20"
 targettime="0" weight="0" /> → PT Jump 20 times
               <exercise id="14" name="Jumping Jack" action="time" targetcount="0"
 targettime="60" weight="0" /> → Jumping Jack 60 seconds
          </group>
          <group id="1" repeat="1" middlerest="60" afterrest="180" mode="none"
``` |
| ```
 numberofexercise="1" order="fixed">
               <exercise id="13" name="Stretching" action="time" targetcount="0"
 targettime="225" weight="0" />
          </group>
          <group id="2" repeat="3" middlerest="60" afterrest="180" mode="manual"
 numberofexercise="2" order="flexible">
               <exercise id="101" name="Leg Extension" action="count"
 targetcount="15" targettime="0" weight="20"/> → Leg Extension, 20Kg 15times
               <exercise id="102" name="Leg Curl" action="count" targetcount="15"
 targettime="0" weight="20"/>
          </group>
          <group id="3" repeat="3" middlerest="0" afterrest="180" mode="auto"
 numberofexercise="2" order="fixed">
               <exercise id="3" name="Rope Jump" action="time/countable"
 targetcount="15" targettime="60" weight="0"/>
               <exercise id="100" name="GymBall Squat" action="count"
 targetcount="12" targettime="0" weight="0"/>
          </group>
          <group id="4" repeat="3" middlerest="60" afterrest="180" mode="manual"
 numberofexercise="1" order="fixed">
               <exercise id="205" name="Crunch" action="countable" targetcount="10"
 targettime="0" weight="0" />
          </group>
     </day>
         <day id="1" numberofgroup="1" currentgroup="0" date="2015-05-05"
 detail="rest">
          <group id="0" repeat="1" middlerest="0" afterrest="0" mode="none"
 numberofexercise="1" order="fixed">
               <exercise id="2" name="Rest Day" action="time" targetcount="0"
 targettime="1800" weight="0"/>
          </group>
``` |
| ```
           ...omitted
..
 </fitnessprogram>
``` |

For example, activity schedule information may include a peripheral device necessary for detecting activity information of a user corresponding to a corresponding exercise and recognition algorithm information like TABLE 8 described below.

**TABLE 8**

| |
|---|
| ```
 <?xml version="1.0" encoding="utf-8"?>
 <exerciselist>
 ...omitted
 <exercise
      id="205"
      name="Crunch"
      muscle="chest"
      difficult="easy"
      metabolism="anaerobic"
      action="count"
      exercise_type="free">
      <information step="4" explain="The exercise is to strengthen the upper part of
 the rectus. It is different from sit-ups in that the waist part is not separated from the
 floor. It is important to maintain tension in the abdomen not only when contracting
 muscles but also when relaxing muscles. "/>
        < engine >
            <device="headset" algorithm_type-'zero_cross" priority="VERY HIGH"
 >
            <device="glass" algorithm_type="zero_cross" priority="VERY HIGH" >
 <device="watch" algorithm_type="zero_cross" priority="HIGH">
 <device="watch" algorithm_type="gravity_coordinate" priority="MID">
 <device="shoes" algorithm_type="gravity_coordinate" priority="MID">
 </engine>
 </exercise>
``` |
| ```
 ...omitted
 </fitnessprogram>
``` |

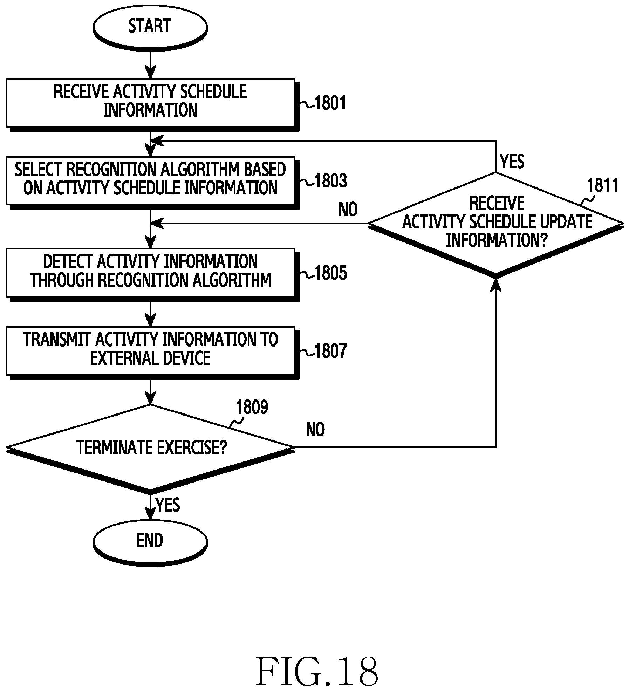

FIG. 18 is a flow-chart of a method for providing an exercise service, based on a control of an external device, by an electronic device, according to an embodiment of the present disclosure. The description below explains an operation for providing an exercise service by the smart watch 100 illustrated in FIG. 1, corresponding to an operation of the smart phone 110 illustrated in FIG. 18. The smart watch 100 of FIG. 1 may include a whole or some of the electronic device 201 of FIG. 2.

Referring to FIG. 18, in operation 1801, an electronic device may receive activity schedule information (e.g., an exercise program) for providing an exercise service. For example, the processor 220 may receive activity schedule information from an external device (e.g., a smart phone 110 of an exercise adviser) controlling an exercise service of the electronic device 201 through the communication interface 270. For example, the processor 220 may receive activity schedule information from a server 206 for an exercise service, through the communication interface 270.

In operation 1803, the electronic device may select one or more recognition algorithms corresponding to activity schedule information among multiple recognition algorithms usable for recognizing activity information of a user. For example, the processor 220 may select one or more recognition algorithms corresponding to the type of exercise included in activity schedule information. For example, the processor 220 may extract recognition algorithm information included in activity schedule information configured in the form of a markup language like TABLE 8. Accordingly, the processor 220 may easily detect activity information of a user for an exercise newly generated by an external device.

In operation 1805, the electronic device may detect activity information of a user through one or more recognition algorithms. For example, the processor 220 may detect activity information of a user by selecting one or more sensors corresponding to one or more recognition algorithms among multiple sensors included in the sensor 280.

In operation 1807, the electronic device may transmit activity information of a user to an external device. For example, the processor 220 may control the communication interface 270 to transmit, to an external device, activity information of a user detected through one or more sensors when an activity information transmission period arrives.

In operation 1809, the electronic device may check whether an exercise of a user is terminated. For example, the processor 220 may check whether an operation of an exercise application is terminated, based on input information detected through the input/output interface 250. For example, the processor 220 may check whether an exercise schedule of a user is terminated, based on activity schedule information.

In operation 1811, the electronic device may check whether activity schedule update information is received from an external device when an exercise of a user has not been terminated. For example, activity schedule update information may include activity schedule information updated by an external device based on activity information of a user.

In operation 1803, the electronic device may select one or more recognition algorithms corresponding to updated activity schedule information when activity schedule update information has been received from an external device.

In operation 1805, the electronic device may detect activity information of a user through one or more recognition algorithms when activity schedule update information has not been received from an external device.

An electronic device may transmit exercise termination information to an external device when an exercise of a user has been terminated. The electronic device may output, to the outside, information on the result of exercise provided from the external device in response to the exercise termination information, through at least one output module. For example, the output module may include at least one of a speaker and a display.

A method of an electronic device may include the operations of: acquiring activity schedule information related to a user activity; selecting one or more recognition algorithms among multiple recognition algorithms for analyzing user activities, at least based on the activity schedule information; analyzing activity information related to the user activity, using the one or more recognition algorithms, thereby providing activity guide information related to the user activity.

A method for selecting one or more sensors from multiple sensors, at least based on the one or more recognition algorithms may be further included.

A method for deactivating one or more sensors that are not selected among the multiple sensors may be further included.

A method for receiving at least a part of the user activity information from an external device functionally connected with the electronic device may be further included.

The multiple recognition algorithms may include a first algorithm that detects a rotational motion, a second algorithm that detects a quantity of change in a movement, a third algorithm that detects a vertical directional movement, a fourth algorithm that detects a horizontal directional movement, a fifth algorithm that detects movement information for correcting other algorithms, or a combination thereof.

The activity schedule information, in the form of a markup language, may include the number of times of exercise, exercise type, exercise time, exercise sequence, exercise intensity, number of repetitions, exercise place related to the user activity, or a combination thereof.

A method for acquiring the activity schedule information may include an operation for acquiring activity schedule information related to the user activity from an external device functionally connected with the electronic device.

A method for acquiring the activity schedule information may include an operation for selecting one piece of activity schedule information from activity schedule information related to multiple user activities stored in a memory of the electronic device, based on a location of the electronic device, weather, time, or a combination thereof.

A method for providing the activity guide information may include an operation for transmitting activity guide information related to the user activity from an external device functionally connected with the electronic device.

The activity guide information may include an exercise quantity, goal achievement rate, posture correction information, biometric information of a user, or a combination thereof.

A method for updating the activity schedule information by analyzing the activity information may be further included.

An electronic device and a method therefor may provide exercise information suitable for a user's physical condition and surrounding environments by configuring or updating activity schedule information (e.g., an exercise program, an exercise schedule, or the like) to correspond to activity information of the user acquired through one or more sensors.

An electronic device and a method therefor may easily acquire activity information of a user corresponding to a new exercise program by recognizing activity information of the user, using multiple recognition algorithms.

At least some of the devices (for example, modules or functions thereof) or the method (for example, operations) may be implemented by a command stored in a non-transitory computer-readable storage medium in a programming module form. When an instruction is implemented by one or more processors (for example, the processor 120), one or more processors may execute a function corresponding to the instruction. The non-transitory computer-readable storage medium may be, for example, the memory 130.

The non-transitory computer readable recoding medium may include a hard disk, a floppy disk, magnetic media (for example, a magnetic tape), optical media (for example, a compact disc read only memory (CD-ROM) and a digital versatile disc (DVD)), magneto-optical media (for example, a floptical disk), a hardware device (for example, a read only memory (ROM), a random access memory (RAM), a flash memory), and the like. In addition, the program instructions may include high class language codes, which can be executed in a computer by using an interpreter, as well as machine codes made by a compiler. Any of the hardware devices as described above may be configured to work as one or more software modules in order to perform the operations according to various embodiments of the present disclosure, and vice versa.

The present disclosure may include a non-transitory computer-readable recording medium in which a program is recorded, the program for executing the operations of selecting one or more recognition algorithms among multiple recognition algorithms for analyzing user activities, and analyzing activity information related to the user activity, using the one or more recognition algorithms, thereby providing activity guide information related to the user activity, at least based on an operation for acquiring activity schedule information related to a user activity and the activity schedule information.

Any of the modules or programming modules may include at least one of the above described elements, exclude some of the elements, or further include other additional elements. The operations performed by the modules, programming module, or other elements may be executed in a sequential, parallel, repetitive, or heuristic manner. Further, some operations may be executed according to another order or may be omitted, or other operations may be added.

While the present disclosure has been shown and described with reference to certain embodiments thereof, it will be understood by those skilled in the art that various changes in form and details may be made therein without departing from the scope of the present disclosure. Therefore, the scope of the present disclosure should not be defined as being limited to the embodiments, but should be defined by the appended claims and equivalents thereof.

## Claims

1. An electronic device (201) for providing a physical exercise service, comprising:
a communication module (270);
a memory (230) configured to store multiple recognition algorithms for analyzing user activities; and
a processor (220) configured to:
acquire activity schedule information related to a physical user activity,
determine at least one recognition algorithm to be used for acquiring physical activity information among the multiple recognition algorithms, at least based on a type of at least one exercise included in the acquired activity schedule information,
acquire status information for each of a plurality of peripheral devices (112, 114, 116, 118, 120) by performing a scanning operation, wherein the status information comprises battery information and supportable recognition algorithm,
determine, based on the determined at least one recognition algorithm and the status information for each of the plurality of peripheral devices, at least one peripheral device among the plurality of peripheral devices,
transmit information of the determined at least one recognition algorithm to the determined at least one peripheral device,
receive physical activity information related to the user activity, from the determined at least one peripheral device, analyze the acquired activity information using the at least one recognition algorithm and
display physical activity guide information related to the user activity based on the analyzed activity information.

2. The electronic device of claim 1, wherein the processor is further configured to acquire the activity schedule information related to the user activity from an external device that is operably connected to the electronic device.

3. The electronic device of claim 1, wherein the processor is further configured to select the activity schedule information related to multiple user activities stored in the memory, based on one of a location of the electronic device, weather, time, and a combination thereof.

4. The electronic device of claim 1, further comprising a communication interface, and wherein
the processor is further configured to transmit activity guide information related to the user activity to an external device that is operably connected to the electronic device, through the communication interface.

5. The electronic device of claim 1, wherein the activity guide information comprises at least one of posture correction information, and exercise sequence change information.

6. A method for providing a physical exercise service, implemented on an electronic device (201), the method comprising:
acquiring activity schedule information related to a physical user activity;
determining at least one recognition algorithm to be used for acquiring physical activity information among multiple recognition algorithms, at least based on a type of at least one exercise included in the acquired activity schedule information;
acquiring status information for each of a plurality of peripheral devices (112, 114, 116, 118, 120) by performing a scanning operation, wherein the status information comprises battery information and supportable recognition algorithm;
determining, based on the determined at least one recognition algorithm and the status information for each of the plurality of peripheral devices, at least one peripheral device among the plurality of peripheral devices;
transmitting information of the determined at least one recognition algorithm to the determined at least one peripheral device;
receiving physical activity information related to the user activity from the determined at least one peripheral device; analyzing the acquired activity information using the at least one recognition algorithm and
displaying physical activity guide information related to the user activity based on the analyzed activity information.

7. The method of claim 6, wherein the multiple recognition algorithms include one of a first algorithm that is configured to detect a rotational motion, a second algorithm that is configured to detect a change in a movement, a third algorithm that is configured to detect a vertical directional movement, a fourth algorithm that is configured to detect a horizontal directional movement, a fifth algorithm that is configured to detect movement information for correcting one of the first through fourth algorithms, and a combination thereof.

8. The method of claim 6, wherein the activity schedule information, which is in the form of a markup language, includes one of a number of times of an exercise, an exercise time, an exercise sequence, an exercise intensity, a number of repetitions, an exercise place related to the user activity, and a combination thereof.

9. The method of claim 6, wherein acquiring the activity schedule information further comprises acquiring the activity schedule information related to the user activity from an external device that is operably connected to the electronic device.

10. The method of claim 6, wherein acquiring the activity schedule information further comprises selecting the activity schedule information from the activity schedule information related to multiple user activities stored in a memory of the electronic device, based on one of a location of the electronic device, weather, time, and a combination thereof.

11. The method of claim 6, wherein providing the activity guide information further comprises transmitting the activity guide information related to the user activity from an external device that is operably connected to the electronic device.

12. The method of claim 6, wherein the activity guide information includes at least one of posture correction information, and exercise sequence change information.

13. The method of claim 6, further comprising
updating the activity schedule information by analyzing the activity information.

## Patentansprüche

1. Elektronische Vorrichtung (201), um einen Service zur körperlichen Betätigung vorzusehen, mit:
einem Kommunikationsmodul (270);
einem Speicher (230), konfiguriert, um mehrfache Erkennungsalgorithmen zu speichern, um Benutzerbetätigungen zu analysieren; und
einem Prozessor (220), konfiguriert, um:
Betätigungszeitplan-Informationen über eine körperliche Betätigung des Benutzers zu erwerben,
mindestens einen Erkennungsalgorithmus zur Erfassung von Informationen zur körperlichen Betätigung unter den mehreren Erkennungsalgorithmen zu bestimmen, mindestens basierend auf einer Art mindestens einer Übung, die in den erfassten Betätigungszeitplan-Informationen inbegriffen ist,
Statusinformationen für jedes aus einer Vielzahl von Peripheriegeräten (112, 114, 116, 118, 120) durch Abtasten zu erwerben, wobei die Statusinformationen Batterieinformationen und unterstützbaren Erkennungsalgorithmus umfassen,
basierend auf dem bestimmten mindestens einen Erkennungsalgorithmus und den Statusinformationen für jedes aus der Vielzahl von Peripheriegeräten mindestens ein Peripheriegerät aus der Vielzahl von Peripheriegeräten zu bestimmen,
Informationen des bestimmten mindestens einen Erkennungsalgorithmus an das bestimmte mindestens eine Peripheriegerät zu übertragen,
Informationen zur körperlichen Betätigung, bezogen auf die Benutzerbetätigung, von dem bestimmten mindestens einen Peripheriegerät zu empfangen, die erfassten Betätigungsinformationen unter Verwendung des mindestens einen Erkennungsalgorithmus zu analysieren und Führungsinformationen zur körperlichen Betätigung, bezogen auf die Benutzerbetätigung, anzuzeigen, basierend auf den analysierten Betätigungsinformationen.

2. Elektronische Vorrichtung nach Anspruch 1, wobei der Prozessor des Weiteren konfiguriert ist, um die Betätigungszeitplan-Informationen über die Benutzerbetätigung von einer externen Vorrichtung zu erwerben, die mit der elektronischen Vorrichtung wirkverbunden ist.

3. Elektronische Vorrichtung nach Anspruch 1, wobei der Prozessor des Weiteren konfiguriert ist, um die Betätigungszeitplan-Informationen über mehrfache Benutzerbetätigungen auszuwählen, die in dem Speicher gespeichert sind, basierend auf einem der Folgenden: einer Position der elektronischen Vorrichtung, Wetter, Zeit und eine Kombination davon.

4. Elektronische Vorrichtung nach Anspruch 1, das des Weiteren eine Kommunikationsschnittstelle umfasst, und wobei
der Prozessor des Weiteren konfiguriert ist, um Betätigungsführungs-Informationen über die Benutzerbetätigung an eine externe Vorrichtung zu übertragen, die mit der elektronischen Vorrichtung wirkverbunden ist, über die Kommunikationsschnittstelle.

5. Elektronische Vorrichtung nach Anspruch 1, wobei die Betätigungs-Führungsinformationen Informationen zur Korrektur der Körperhaltung und/oder Informationen zur Änderung des Übungsablaufs umfassen.

6. Verfahren zur Bereitstellung eines Service zur körperlichen Betätigung, der auf einer elektronischen Vorrichtung (201) implementiert ist, wobei das Verfahren Folgendes umfasst:
Erfassung von Betätigungszeitplan-Informationen bezogen auf eine körperliche Betätigung des Benutzers;
Bestimmung mindestens eines Erkennungsalgorithmus zur Erfassung von Informationen zur körperlichen Betätigung unter mehreren Erkennungsalgorithmen, mindestens basierend auf einem Typ von mindestens einer Übung, die in den erfassten Betätigungszeitplan-Informationen enthalten ist;
Erfassung von Statusinformationen für jedes aus einer Vielzahl von Peripheriegeräten (112, 114, 116, 118, 120) durch Abtasten, wobei die Statusinformationen Batterieinformationen und unterstützbaren Erkennungsalgorithmus umfassen;
Bestimmung, basierend auf dem bestimmten mindestens einen Erkennungsalgorithmus und den Statusinformationen für jedes aus der Vielzahl von Peripheriegeräten, mindestens eines Peripheriegeräts unter der Vielzahl von Peripheriegeräten;
Übertragung von Informationen des bestimmten mindestens einen Erkennungsalgorithmus an das bestimmte mindestens eine Peripheriegerät;
Empfang von Informationen zur körperlichen Betätigung, bezogen auf die Benutzerbetätigung, von dem bestimmten mindestens einen Peripheriegerät, Analyse der erfassten Betätigungsinformationen unter Verwendung des mindestens einen Erkennungsalgorithmus und Anzeige von Führungsinformationen zur körperlichen Betätigung, bezogen auf die Benutzerbetätigung, basierend auf den analysierten Betätigungsinformationen.

7. Verfahren nach Anspruch 6, wobei die mehreren Erkennungsalgorithmen eines der folgenden umfassen: ein erster Algorithmus, der konfiguriert ist, um eine Rotationsbewegung zu ermitteln, ein zweiter Algorithmus, der konfiguriert ist, um eine Veränderung in einer Bewegung zu ermitteln, ein dritter Algorithmus, der konfiguriert ist, um eine senkrechte Richtungsbewegung zu ermitteln, ein vierter Algorithmus, der konfiguriert ist, um eine waagrechte Richtungsbewegung zu ermitteln, ein fünfter Algorithmus, der konfiguriert ist, um Bewegungsinformationen zu ermitteln, um einen der ersten bis vierten Algorithmen zu korrigieren, und eine Kombination davon.

8. Verfahren nach Anspruch 6, wobei die Betätigungszeitplan-Informationen, die die Form einer Auszeichnungssprache haben, eines der Folgenden umfassen: die Häufigkeit einer Übung, eine Übungszeit, einen Übungsablauf, eine Übungsintensität, eine Zahl von Wiederholungen, einen Übungsort bezogen auf die Benutzerbetätigung, und eine Kombination davon.

9. Verfahren nach Anspruch 6, wobei die Erfassung der Betätigungszeitplan-Informationen des Weiteren die Erfassung der Betätigungszeitplan-Informationen über die Benutzerbetätigung von einer externen Vorrichtung her umfassen, die mit der elektronischen Vorrichtung wirkverbunden ist.

10. Verfahren nach Anspruch 6, wobei die Erfassung der Betätigungszeitplan-Informationen des Weiteren die Auswahl der Betätigungszeitplan-Informationen aus den Betätigungszeitplan-Informationen über mehrere Benutzerbetätigungen umfasst, die in einem Speicher der elektronischen Vorrichtung gespeichert sind, basierend auf einem der Folgenden: einer Position der elektronischen Vorrichtung, Wetter, Zeit und einer Kombination davon.

11. Verfahren nach Anspruch 6, wobei die Bereitstellung der Betätigungs-Führungsinformationen des Weiteren die Übertragung der Betätigungs-Führungsinformationen über die Benutzerbetätigung von einer externen Vorrichtung her umfasst, die mit der elektronischen Vorrichtung wirkverbunden ist.

12. Verfahren nach Anspruch 6, wobei die Betätigungs-Führungsinformationen Informationen zur Korrektur der Körperhaltung und/oder Informationen zur Änderung des Übungsablaufs umfassen.

13. Verfahren nach Anspruch 6, das des Weiteren die Aktualisierung der Betätigungszeitplan-Informationen durch Analyse der Betätigungsinformationen umfasst.

## Revendications

1. Dispositif électronique (201) pour fournir un service d'exercice physique, comprenant :
un module de communication (270) ;
une mémoire (230) configurée pour stocker de multiples algorithmes de reconnaissance pour analyser des activités de l'utilisateur ; et
un processeur (220) configuré pour :
acquérir des informations de programme d'activité associées à une activité physique de l'utilisateur,
déterminer au moins un algorithme de reconnaissance à utiliser pour acquérir des informations d'activité physique parmi les multiples algorithmes de reconnaissance , au moins sur la base d'un type d'au moins un exercice inclus dans les informations de programme d'activité acquises,
acquérir des informations d'état pour chacun d'une pluralité de dispositifs périphériques (112, 114, 116, 118, 120) en effectuant une opération de balayage, où les informations d'état comprennent des informations de batterie et un algorithme de reconnaissance supportable,
déterminer, sur la base de l'au moins un algorithme de reconnaissance déterminé et des informations d'état pour chacun de la pluralité de dispositifs périphériques, au moins un dispositif périphérique parmi la pluralité de dispositifs périphériques,
transmettre des informations de l'au moins un algorithme de reconnaissance déterminé à l'au moins un dispositif périphérique déterminé,
recevoir des informations d'activité physique associées à l'activité de l'utilisateur, à partir de l'au moins un dispositif périphérique déterminé, analyser les informations d'activité acquises en utilisant l'au moins un algorithme de reconnaissance et
afficher des informations de guide d'activité physique associées à l'activité de l'utilisateur sur la base des informations d'activité analysées.

2. Dispositif électronique selon la revendication 1, où le processeur est configuré en outre pour acquérir les informations de programme d'activité associées à l'activité de l'utilisateur à partir d'un dispositif externe qui est connecté de façon opérationnelle au dispositif électronique.

3. Dispositif électronique selon la revendication 1, où le processeur est configuré en outre pour sélectionner les informations de programme d'activité associées à de multiples activités de l'utilisateur stockées dans la mémoire, sur la base d'une parmi la position du dispositif électronique, les conditions climatiques, l'heure, et une combinaison de celles-ci.

4. Dispositif électronique selon la revendication 1, comprenant en outre une interface de communication, et où
le processeur est configuré en outre pour transmettre des informations de guide d'activité associées à l'activité de l'utilisateur à un dispositif externe qui est connecté de façon opérationnelle au dispositif électronique, à travers l'interface de communication.

5. Dispositif électronique selon la revendication 1, où les informations de guide d'activité comprennent au moins une parmi des informations de correction de posture et des informations de changement de séquence d'exercices.

6. Procédé pour fournir un service d'exercice physique, implémenté sur un dispositif électronique (201), le procédé comprenant :
acquérir des informations de programme d'activité associées à une activité physique de l'utilisateur ;
déterminer au moins un algorithme de reconnaissance à utiliser pour acquérir des informations d'activité physique parmi de multiples algorithmes de reconnaissance, au moins sur la base d'un type d'au moins un exercice inclus dans les informations de programme d'activité acquises ;
acquérir des informations d'état pour chacun d'une pluralité de dispositifs périphériques (112, 114, 116, 118, 120) en effectuant une opération de balayage, où les informations d'état comprennent des informations de batterie et un algorithme de reconnaissance supportable ;
déterminer, sur la base de l'au moins un algorithme de reconnaissance déterminé et des informations d'état pour chacun de la pluralité de dispositifs périphériques, au moins un dispositif périphérique parmi la pluralité de dispositifs périphériques ;
transmettre des informations de l'au moins un algorithme de reconnaissance déterminé à l'au moins un dispositif périphérique déterminé ;
recevoir des informations d'activité physique associées à l'activité de l'utilisateur provenant de l'au moins un dispositif périphérique déterminé ; analyser les informations d'activité acquises en utilisant l'au moins un algorithme de reconnaissance et
afficher des informations de guide d'activité physique associées à l'activité de l'utilisateur sur la base des informations d'activité analysées.

7. Procédé selon la revendication 6, où les multiples algorithmes de reconnaissance comprennent l'un parmi un premier algorithme qui est configuré pour détecter un mouvement de rotation, un deuxième algorithme qui est configuré pour détecter un changement dans un mouvement, un troisième algorithme qui est configuré pour détecter un mouvement directionnel vertical, un quatrième algorithme qui est configuré pour détecter un mouvement directionnel horizontal, un cinquième algorithme qui est configuré pour détecter des informations de mouvement pour corriger l'un des premier à quatrième algorithmes, et une combinaison de ceux-ci.

8. Procédé selon la revendication 6, où les informations de programme d'activité, qui se présentent sous la forme d'un langage de balisage, comprennent l'un parmi un nombre de fois d'un exercice, une durée d'exercice, une séquence d'exercice, une intensité d'exercice, un nombre de répétitions, un lieu d'exercice associé à l'activité de l'utilisateur, et une combinaison de ceux-ci.

9. Procédé selon la revendication 6, où l'acquisition des informations de programme d'activité comprend en outre l'acquisition des informations de programme d'activité associées à l'activité de l'utilisateur à partir d'un dispositif externe qui est connecté de façon opérationnelle au dispositif électronique.

10. Procédé selon la revendication 6, où l'acquisition des informations de programme d'activité comprend en outre la sélection des informations de programme d'activité parmi les informations de programme d'activité associées à de multiples activités de l'utilisateur stockées dans une mémoire du dispositif électronique, sur la base d'une parmi une position du dispositif électronique, les conditions climatiques, l'heure, et une combinaison de celles-ci.

11. Procédé selon la revendication 6, où la fourniture des informations de guide d'activité comprend en outre la transmission des informations de guide d'activité associées à l'activité de l'utilisateur à partir d'un dispositif externe qui est connecté de façon opérationnelle au dispositif électronique.

12. Procédé selon la revendication 6, où les informations de guide d'activité comprennent au moins une parmi des informations de correction de posture et des informations de changement de séquence d'exercices.

13. Procédé selon la revendication 6, comprenant en outre la mise à jour des informations de programme d'activité en analysant les informations d'activité.
